# EUROPEAN PATENT SPECIFICATION

(11) **EP 3 746 088 B1**
(45) Date of publication and mention of the grant of the patent: **10.07.2024**
(21) Application number: 19748292.0
(22) Date of filing: 01.02.2019
(51) Int. Cl.: A61K 31/7088, A61K 45/00, A61K 48/00, A61P 35/00, C12N 15/117

(54) **COMPOSITIONS AND METHODS FOR INDUCING TRIPARTITE MOTIF-CONTAINING PROTEIN 16 (TRIM16) SIGNALING**
ZUSAMMENSETZUNGEN UND VERFAHREN ZUR INDUKTION DER SIGNALISIERUNG DES PROTEINS 16 MIT DREITEILIGEM MOTIV (TRIM16)
COMPOSITIONS ET PROCÉDÉS POUR INDUIRE UNE SIGNALISATION DÉPENDANTE DE LA PROTÉINE 16 (TRIM16)

(30) Priority: 02.02.2018 US 201862625623 P
(43) Date of publication of application: 09.12.2020
(73) Proprietor: University of Washington, Seattle, WA 98105-4721 (US)
(72) Inventor: GALE, Michael, J., Jr., Seattle, WA 98105-4721 (US)
(74) Representative: Gevers Patents
(86) International application number: PCT/US2019/016413
(87) International publication number: WO 2019/152884

(56) References cited:
- WO-A2-2016/179034
- US-A1- 2014 037 716
- US-A1- 2015 017 207
- US-A1- 2015 017 207
- US-A1- 2017 246 281
- LI-WEN LIU ET AL: "An RNA Molecule Derived From Sendai Virus DI Particles Induces Antitumor Immunity and Cancer Cell-selective Apoptosis", MOLECULAR THERAPY, vol. 24, no. 1, 1 January 2016 (2016-01-01), US, pages 135 - 145, XP055725036, ISSN: 1525-0016, DOI: 10.1038/mt.2015.201
- KIM PATRICK Y. ET AL: "TRIM16 overexpression induces apoptosis through activation of caspase-2 in cancer cells", APOPTOSIS, vol. 18, no. 5, 1 May 2013 (2013-05-01), GB, pages 639 - 651, XP093013143, ISSN: 1360-8185, Retrieved from the Internet <URL:https://www.ncbi.nlm.nih.gov/pmc/articles/PMC3618413/pdf/10495_2013_Article_813.pdf> DOI: 10.1007/s10495-013-0813-y
- VERSTEEG, GA ET AL.: "InTRIMsic immunity: Positive and negative regulation of immune signaling by tripartite motif proteins", CYTOKINE AND GROWTH FACTOR REVIEWS, vol. 25, no. 5, October 2014 (2014-10-01), pages 563 - 576, XP029090049, [retrieved on 20140813], doi:10.1016/j.cytogfr.2014.08.001
- KELL, A ET AL.: "Pathogen-Associated Molecular Pattern Recognition of Hepatitis C Virus Transmitted/Founder Variants by RIG-I Is Dependent on U-Core Length", JOURNAL OF VIROLOGY, vol. 89, no. 21, November 2015 (2015-11-01), pages 11056 - 11068, XP055628303, ISSN: 0022-538X, [retrieved on 20150826], DOI: 10.1128/JVI.01964-15
- TURRINI, F ET AL.: "HIV-1 transcriptional silencing caused by TRIM22 inhibition of Sp1 binding to the viral promoter", RETROVIROLOGY, vol. 12, no. 104, 18 December 2015 (2015-12-18), pages 1 - 8, XP055628306, DOI: 10.1186/s12977-015-0230-0
- UCHIL, PD ET AL.: "TRIM E3 Ligases Interfere with Early and Late Stages of the Retroviral Life Cycle", PLOS PATHOGENS, vol. 4, no. 2, 8 February 2008 (2008-02-08), pages 1 - 13, XP055628308, DOI: 10.1371/journal.ppat.0040016
- KAWAI, T ET AL.: "Regulation of innate immune signalling pathways by the tripartite motif (TRIM) family proteins", EMBO MOLECULAR MEDICINE, vol. 3, no. 9, September 2011 (2011-09-01), pages 513 - 527, XP055628309, ISSN: 1757-4676, DOI: 10.1002/emmm.201100160

## Description

### BACKGROUND

Cancers are characterized by a cell's loss of the ability to control the cell cycle, resulting in abnormal and uncontrolled cell growth. The transformed cells often lose the ability to regulate their division and growth, as well as to respond to typical signals that induce programmed cell death (PCD) in healthy cells. Traditional strategies to treat cancers include surgery to remove cancerous tissues (e.g., tumors) and administration of toxic compositions that predominantly affect the cancer cells in the body. Great strides have been made in the development of immunotherapeutics that leverage compositions and/or signaling pathways of the subject's immune system to attack cancer cells. Such therapeutic approaches include, for example, administration of cancer-specific antibodies containing toxic payloads, administration of checkpoint inhibitors that prevent cancer cells from blocking or preventing host responses against the cancer cells, and adoptive cell therapies, including CAR T approaches that use altered immune cells to specifically initiate and target host immune responses to the cancer cells.

However, notwithstanding the many advances in anti-cancer therapies, issues remain relating to toxic side-effects, resistance of cancer cells, recurrence of disease, and unaffordability and inaccessibility of many treatments. Accordingly, a need remains for cancer treatments that promote the death of the cells with minimal off-site toxicity. Furthermore, to ensure accessibility of healthcare and reduction of attendant costs, a need remains for treatments that can be broadly applied to many types of cancers and do not require personalization on a case to case basis.

US 2015/017207 discloses methods for RIG-I activation using a pathogen associated molecular pattern (PAMP) containing nucleic acid molecule.

This document is silent on cancer.

LI-WEN LIU et al, "an RNA Molecule Derived From Sendai Virus DI Particles Induces Antitumor Immunity and Cancer Cell-selective Apoptosis", MOLECULAR THERAPY, vol. 24, n°1, 1 January 2016 (2016-01-01), pages 135-145, teaches that the double-standed stem and presence of 5'-triphosphate in Sendai viral PAMP RNA are essential for the induction of cancer cell-specific apoptosis.

The present invention addresses these and related needs in providing a composition for use in the treatment of cancer in a subject comprising a combination of pathogen associated molecular pattern (PAMP) containing nucleic acid molecule described herein and a nucleic acid encoding TRIM16 operatively linked to a promoter

### SUMMARY

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

In one aspect, the description discloses a method of inducing tripartite motif-containing protein 16 (TRIM16) activity in a cell. The method comprises contacting the cell with an effective amount of a pathogen associated molecular pattern (PAMP) containing nucleic acid molecule. The PAMP containing nucleic acid molecule comprises: a 5' arm region comprising a terminal triphosphate, a poly uracil core comprising at least 8 contiguous uracil residues, and a 3' arm region comprising at least 8 nucleic acid residues. The 5' most nucleic acid residue of the 3' arm region is not a uracil and wherein the 3' arm region is at least 30% uracil residues.

In some embodiments, the poly uracil core consists of between 8 and 30 uracil residues. In some embodiments, the 5' most nucleic acid residue of the 3' arm region is a cytosine residue or a guanine residue. In some embodiments, the 3' arm region is at least 40%, 50%, 60%, 70%, 80%, or 90% uracil residues. In some embodiments, the 3' arm region comprises at least 7 contiguous uracil residues. In some embodiments, the 5' arm region further comprises one or more nucleic acid residues disposed between the terminal triphosphate and the poly uracil core. In some embodiments, the 5' arm region consists of the terminal triphosphate, and wherein the terminal triphosphate is linked directly to the 5'-end of the poly uracil core. In some embodiments, the terminal triphosphate, the one or more nucleic acid residues of the 5' arm region, and the poly uracil core do not naturally occur together in a Hepatitis C virus.

In some embodiments, the PAMP containing nucleic acid molecule induces retinoic acid-inducible gene I (RIG-I) like receptor (RLR) interaction with TRIM16 in the cell. In some embodiments, the RLR interaction with TRIM16 induces cell death signaling in the cell. In some embodiments, the RLR is RIG-I.

In some embodiments, the method further comprises contacting the cell with interferon (IFN). In some embodiments, the method further comprises contacting the cell with a nucleic acid comprising a sequence encoding TRIM16 operatively linked to a promoter sequence, wherein the promoter sequence is capable of inducing expression of the encoded TRIM16 in the cell. In some embodiments, the encoded TRIM16 has an amino acid sequence of at least 90% identity to the amino acid sequence set forth in SEQ ID NO: 1.

In some embodiments, TRIM16 activity comprises induction of programmed cell death.

In some embodiments, the cell is a cancer cell. In some embodiments, the cancer cell is a solid tumor cancer cell. In some embodiments, the cancer cell is a solid tumor cancer cell selected from pancreatic cancer, bladder cancer, colorectal cancer, breast cancer, prostate cancer, renal cancer, hepatocellular cancer, lung cancer, ovarian cancer, cervical cancer, gastric cancer, esophageal cancer, head and neck cancer, melanoma, neuroendocrine cancers, CNS cancers, brain tumors, bone cancer, and soft tissue sarcoma. In some embodiments, the cancer cell is a hematological cancer cell.

In some embodiments, the cell is contacted with the effective amount of the PAMP containing nucleic acid molecule *in vitro.* In some embodiments, the cell is contacted with the effective amount of the PAMP containing nucleic acid molecule *in vivo* in a mammalian subject. In some embodiments, the method further comprises administering to the subject an immunotherapy for the cancer. In some embodiments, the immunotherapy comprises cancer-specific antibodies or functional fragments thereof, immune checkpoint inhibitors, and adoptive cell therapies, including CAR T cell therapy. In some embodiments, the subject is a human.

In another aspect, the description discloses a method of treating cancer in a subject in need thereof. The method comprises administering to the subject a therapeutically effective amount of a pathogen associated molecular pattern (PAMP) containing nucleic acid molecule. The PAMP containing nucleic acid molecule comprises: a 5' arm region comprising a terminal triphosphate, a poly uracil core comprising at least 8 contiguous uracil residues, and a 3' arm region comprising at least 8 nucleic acid residues. The 5' most nucleic acid residue of the 3' arm region is not a uracil and wherein the 3' arm region is at least 30% uracil residues.

In some embodiments, the poly uracil core consists of between 8 and 30 uracil residues. In some embodiments, the 5' most nucleic acid residue of the 3' arm region is a cytosine residue or a guanine residue. In some embodiments, the 3' arm region is at least 40%, 50%, 60%, 70%, 80%, or 90% uracil residues. In some embodiments, the 3' arm region comprises at least 7 contiguous uracil residues. In some embodiments, the 5' arm region further comprises one or more nucleic acid residues disposed between the terminal triphosphate and the poly uracil core. In some embodiments, the 5' arm region consists of the terminal triphosphate, and wherein the terminal triphosphate is linked directly to the 5'-end of the poly uracil core. In some embodiments, the terminal triphosphate, the one or more nucleic acid residues of the 5' arm region, and the poly uracil core do not naturally occur together in a Hepatitis C virus.

In some embodiments, the PAMP containing nucleic acid molecule induces retinoic acid-inducible gene I (RIG-I) like receptor (RLR) interaction with TRIM16 in a cancer cell in the subject. In some embodiments, the RLR interaction with TRIM16 induces cell death signaling in the cancer cell. In some embodiments, the RLR is RIG I.

In some embodiments, the method further comprises administering an effective amount of interferon (IFN) to the subject. In some embodiments, the method further comprises administering to the subject a nucleic acid comprising a sequence encoding TRIM16 operatively linked to a promoter sequence, wherein the promoter sequence is capable of inducing expression of the encoded TRIM16 in a cancer cell within the subject.

In some embodiments, the cancer is a solid tumor cancer selected from pancreatic cancer, bladder cancer, colorectal cancer, breast cancer, prostate cancer, renal cancer, hepatocellular cancer, lung cancer, ovarian cancer, cervical cancer, gastric cancer, esophageal cancer, head and neck cancer, melanoma, neuroendocrine cancers, CNS cancers, brain tumors, bone cancer, and soft tissue sarcoma. In some embodiments, the cancer is a hematological cancer.

In some embodiments, the method further comprises administering to the subject an immunotherapy for the cancer. In some embodiments, the immunotherapy comprises cancer-specific antibodies or functional fragments thereof, immune checkpoint inhibitors, and adoptive cell therapies, including CAR T cell therapy. In some embodiments, the subject is a human.

In another aspect, the description discloses a therapeutic composition comprising a combination of pathogen associated molecular pattern (PAMP) containing nucleic acid molecule described herein and interferon (IFN). In some embodiments, the IFN is type 1 IFN. In some embodiments, the IFN is pegylated IFN. In some embodiments, the IFN is IFN lambda. In some embodiments, the composition further comprises a nucleic acid encoding TRIM16 operatively linked to a promoter. In some embodiments, the composition is formulated in a liposome.

The invention provides a therapeutic composition comprising a combination of pathogen associated molecular pattern (PAMP) containing nucleic acid molecule described herein and a nucleic acid encoding TRIM16 operatively linked to a promoter. In some embodiments, the composition further comprises interferon (IFN). In some embodiments, the IFN is type 1 IFN. In some embodiments, the IFN is pegylated IFN. In some embodiments, the composition is formulated in a liposome.

### DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:

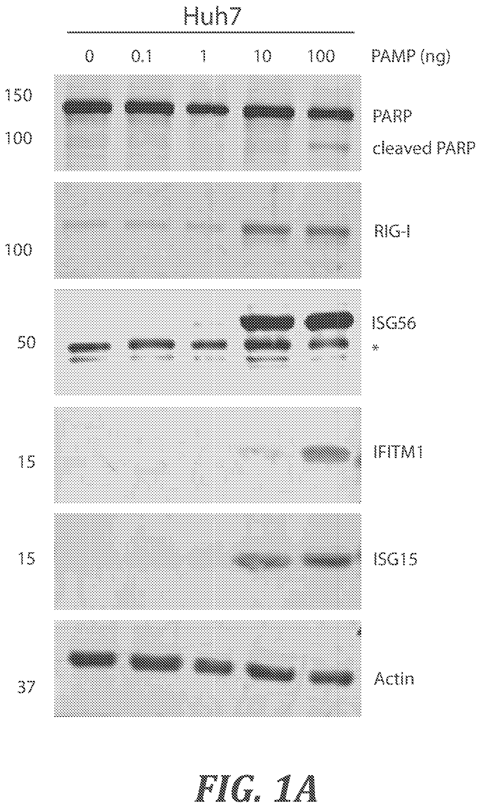

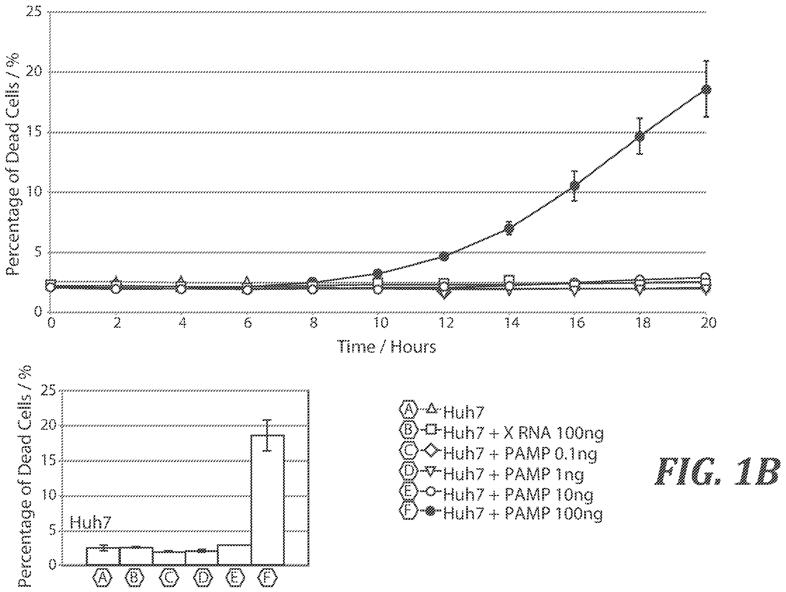

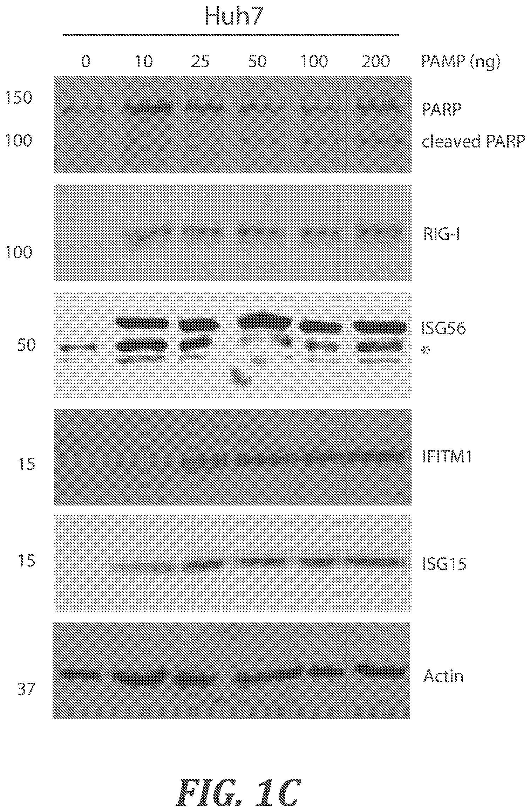

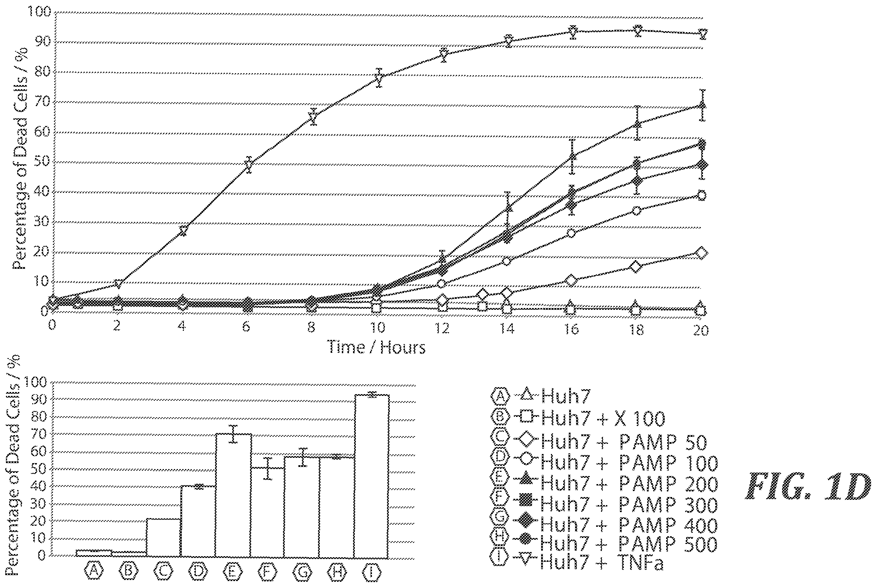

FIGURES 1A-1D illustrate that PAMP RNA induces innate immune signaling and caspase activity in a dose-dependent manner, but at different dose ranges. FIGURE 1A is a western blot showing dose-dependent activation of innate immune signaling in human hepatocellular carcinoma (Huh7) cells at all doses of HCV PAMP RNA, and a dose-dependent induction of apoptosis. FIGURE 1B graphically illustrates cell death analysis that reveals an induction of cell death corresponding to the activation of caspase activity. Bar graph shows the percentage of cell death after 20h. FIGURE 1C is a western blot revealing a differential threshold for PAMP RNA-induced activation of innate immune signaling and caspase activation. FIGURE 1D graphically illustrates cell death analysis that reveals a dose-dependent increase in cell death induction up to a threshold of 200ng. Bar graph shows the percentage of cell death after 20h.

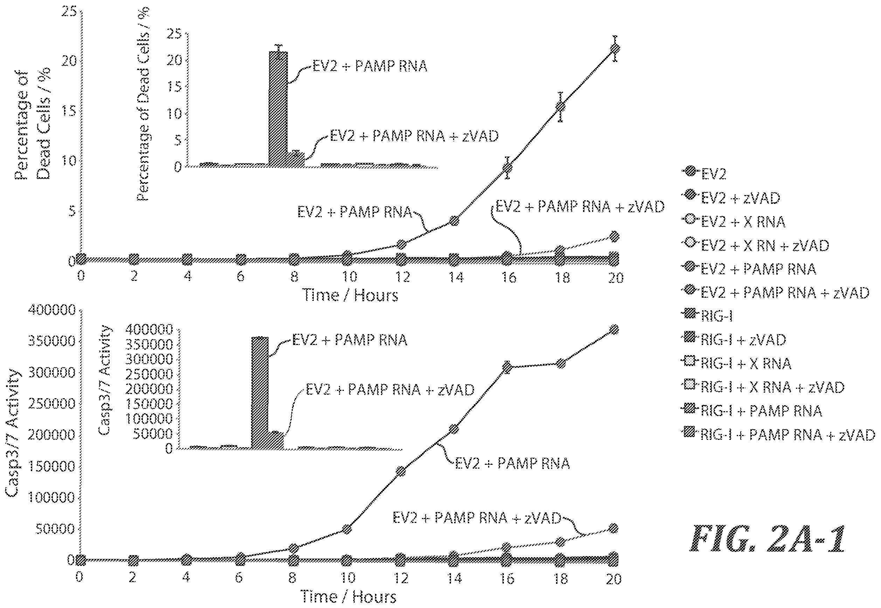

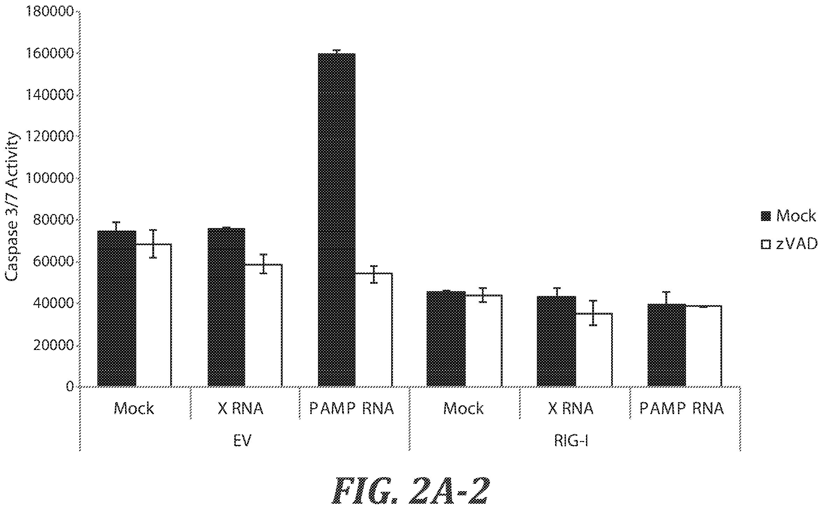

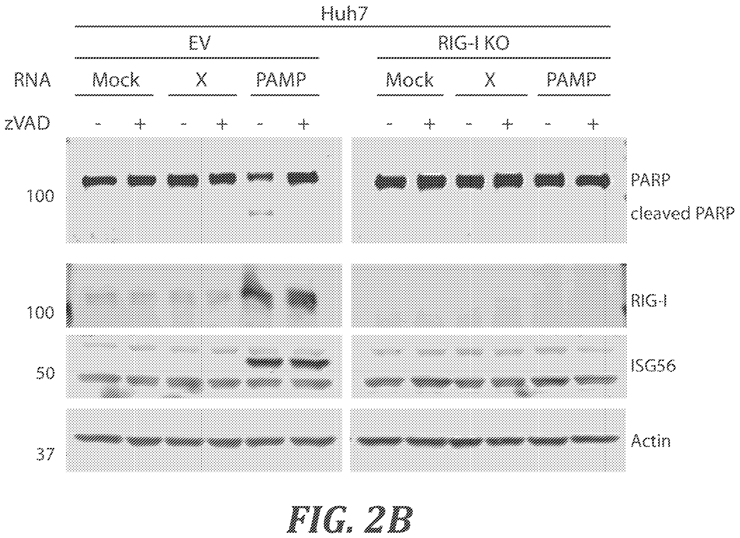

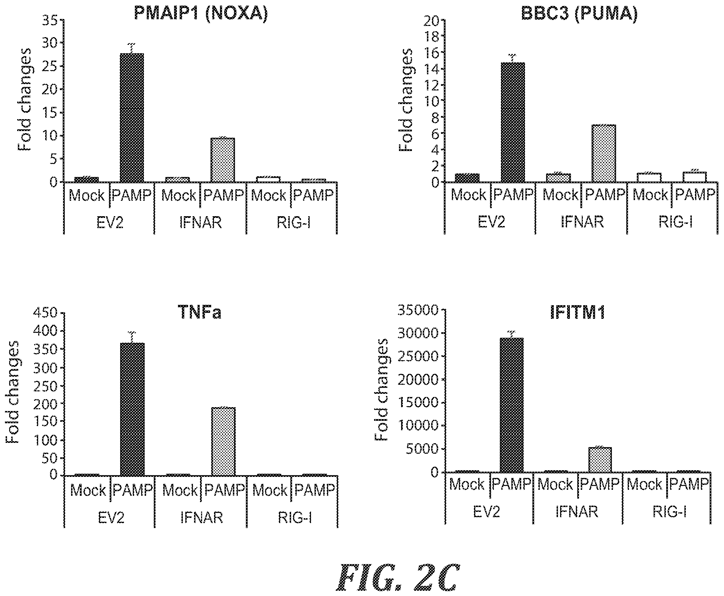

FIGURES 2A-2C illustrate that HCV PAMP RNA induction of apoptosis is dependent on RIG-I and activated through the classical caspase-dependent pathway. FIGURE 2A graphically illustrates that knocking out RIG-I rescues the induction of apoptosis by HCV PAMP RNA (FIGURE 2A-1). Apoptosis is induced by RIG-I-dependent caspase activation and apoptosis is abrogated by treatment with zVAD, a pan-caspase inhibiting compound (FIGURE 2A-2). Compared to mock,* P < 0.05 and **** P < 0.0001. FIGURE 2B is a Western blot that illustrates that treatment with zVAD rescues HCV PAMP RNA-induced apoptosis but does not alter its activation of the innate immune response. FIGURE 2C graphically illustrates that the absence of RIG-I abrogates the transcriptional activation of the p53-dependent pro-apoptotic genes NOXA and PUMA in response to HCV PAMP RNA.

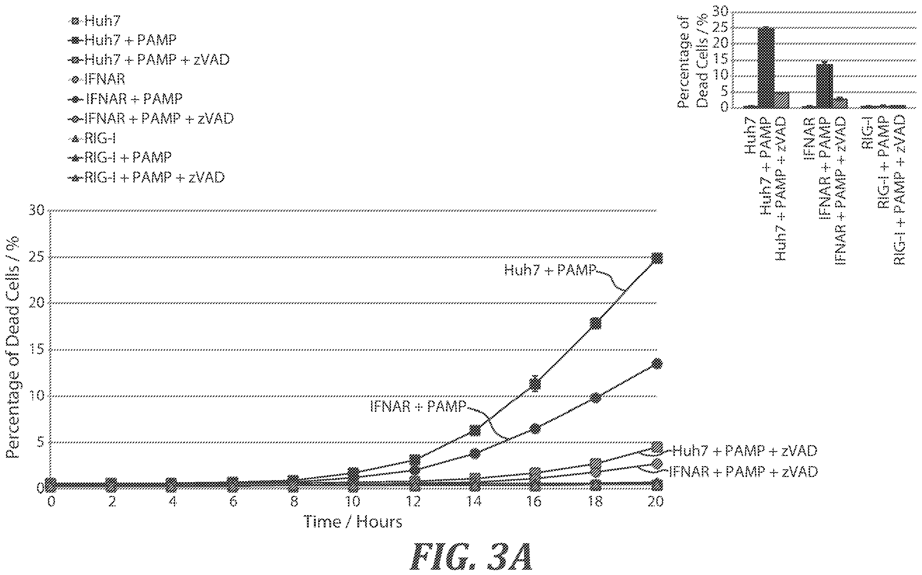

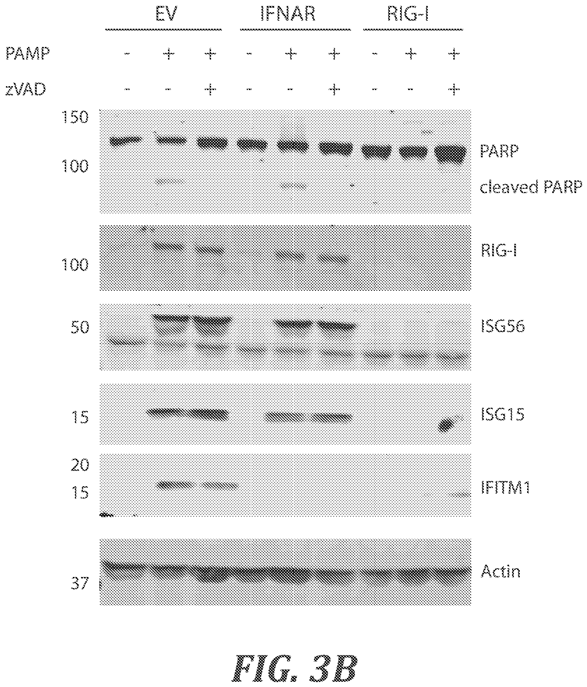

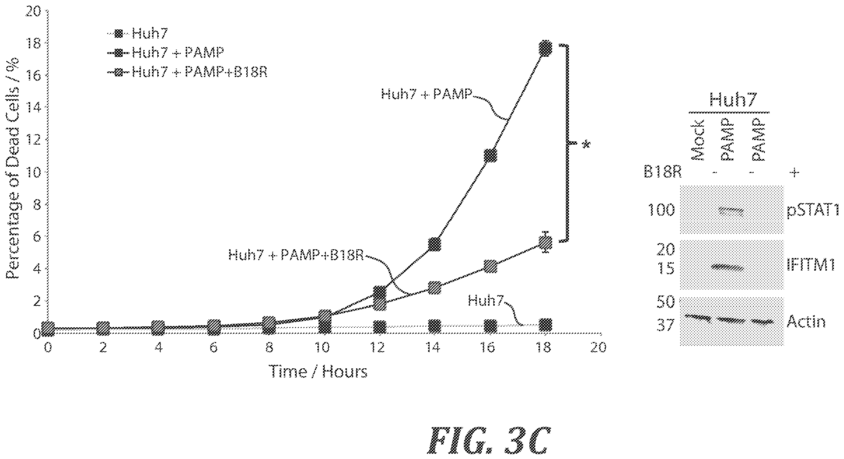

FIGURES 3A-3C illustrate that the type I IFN pathway plays a role in RIG-I-mediated induction of apoptosis. FIGURE 3A graphically illustrates that knock down of the type I IFN receptor chain, IFNAR1, partially abrogates RIG-I-mediated induction of apoptosis. FIGURE 3B is a western blot that shows the induction of the innate immune response is impaired in both IFNAR and RIG-I KO cells. FIGURE 3C illustrates that blocking type I IFN signaling with B 18R, a purified protein inhibitor of IFNAR signaling that is encoded by vaccinia virus, leads to an impairment of RIG-I-mediated induction of apoptosis (left). A western blot reveals the blockage of type I IFN signaling by B18R (right). Compared to Huh7 + PAMP,* P < 0.05.

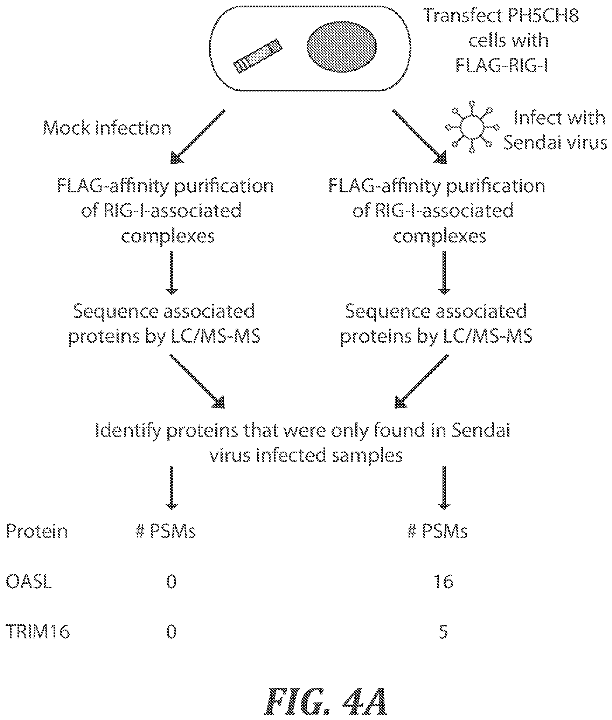

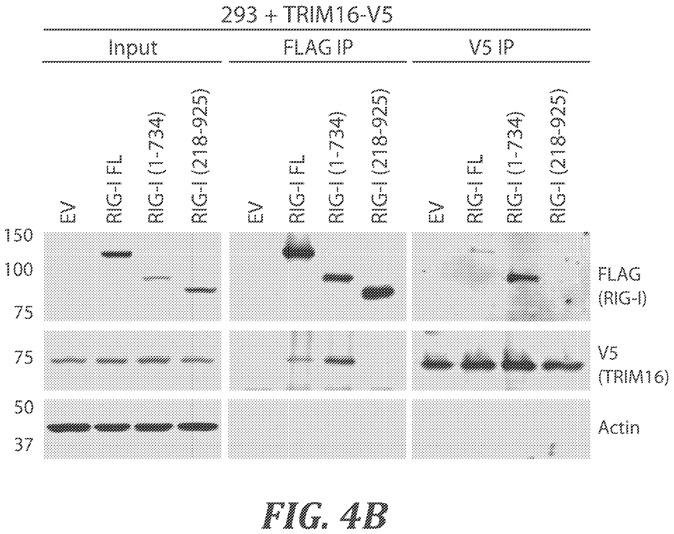

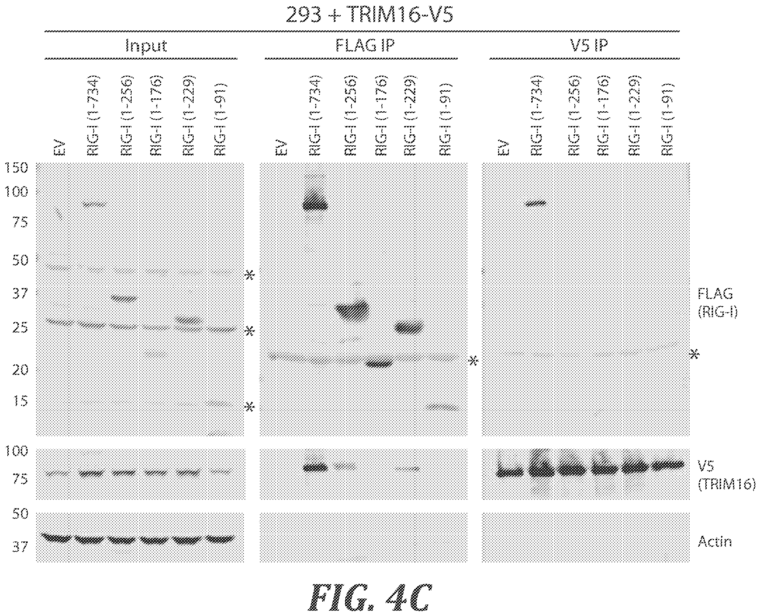

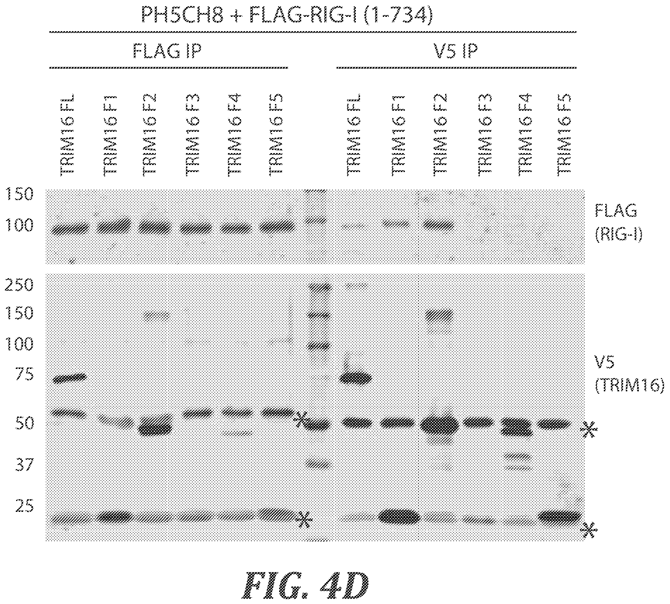

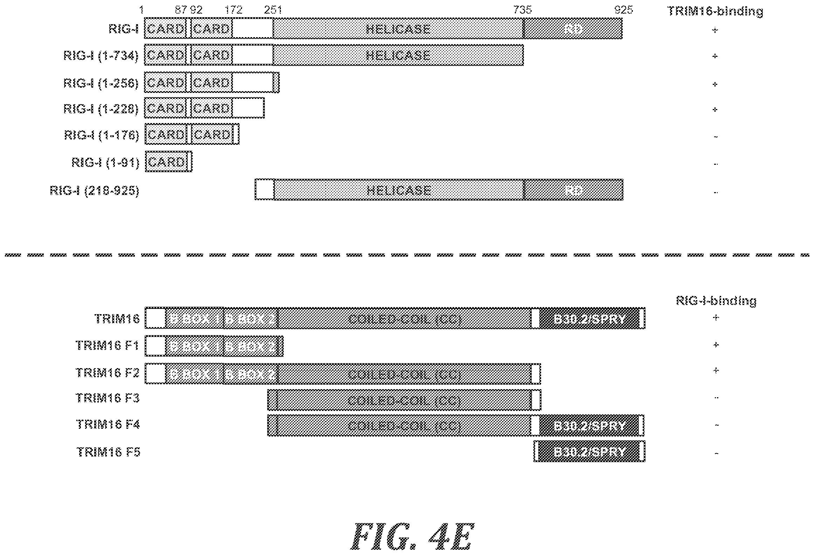

FIGURES 4A-4E illustrate that TRIM16 is a novel interactor of RIG-I. FIGURE 4A schematically illustrates that work flow to identify proteins to bind to RIG-I after activation by Sendai virus. FIGURE 4B shows a Western blot that illustrates that the N-terminal of RIG-I is necessary for binding to TRIM16. FIGURE 4C shows a Western blot that illustrates that the CARD domains of RIG-I are necessary and sufficient for binding to TRIM16. FIGURE 4D shows a Western blot that illustrates that the N-terminal B-Box domains of TRIM16 are necessary and sufficient for binding to RIG-I. * denotes non-specific bands. FIGURE 4B is a cartoon depicting a summary of the interaction between RIG-I and TRIM16.

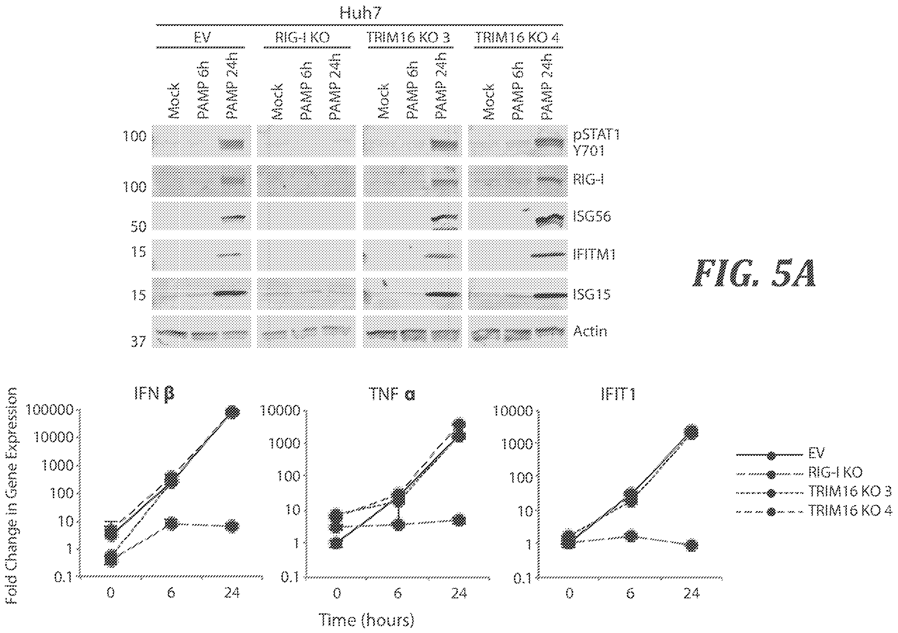

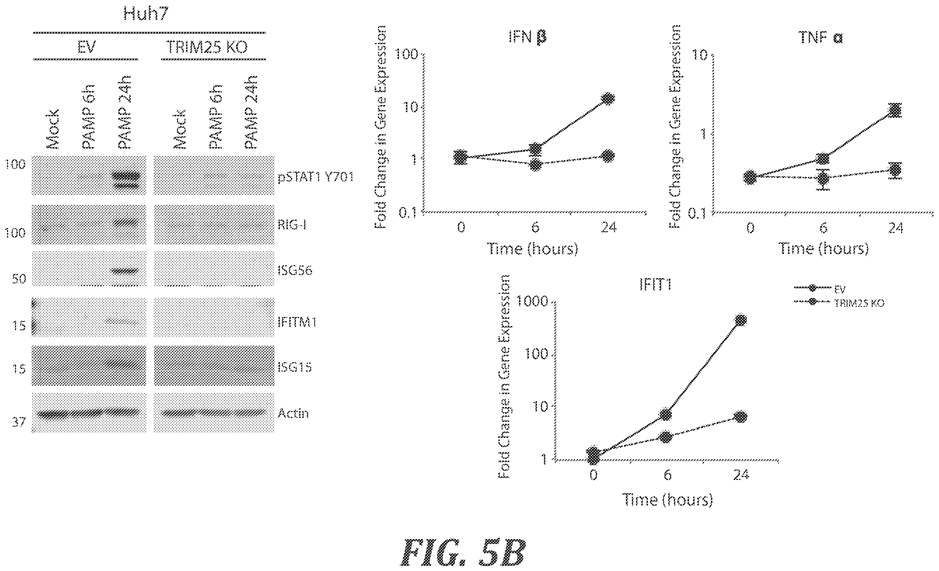

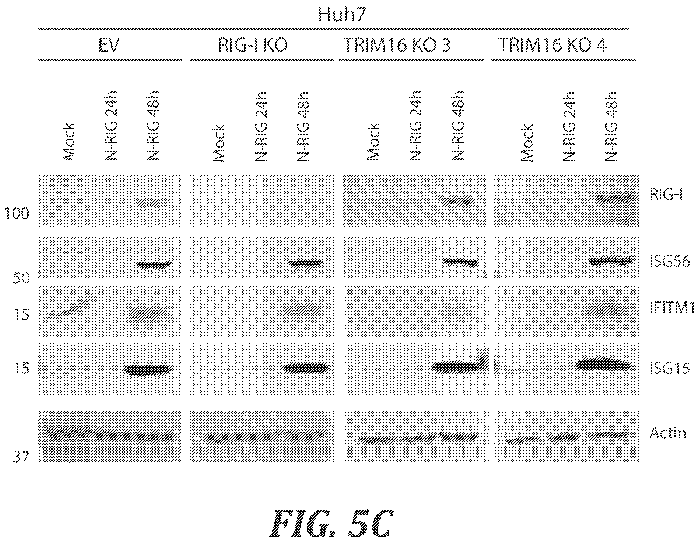

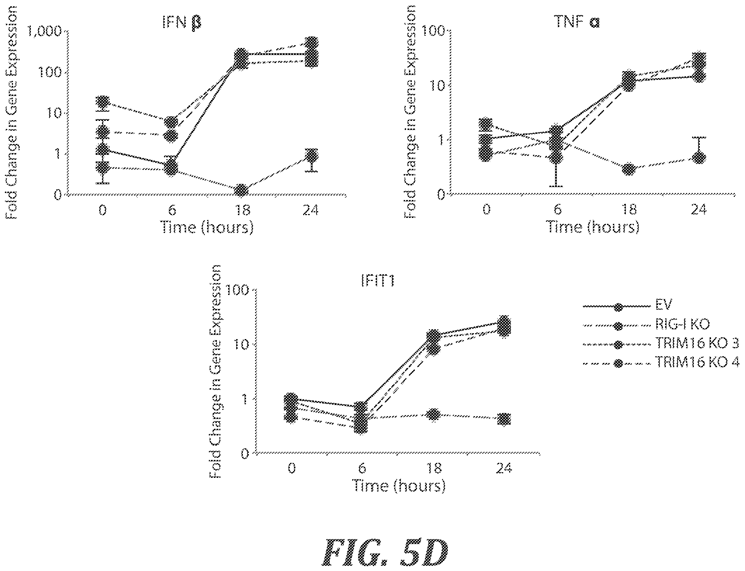

FIGURES 5A-5D illustrate that TRIM16 is not involved in the host innate immune response. FIGURE 5A shows a Western blot that illustrates that knock out of TRIM16 does not have an effect on PAMP-mediated innate immune response. FIGURE 5B is a Western blot that illustrates that, in contrast, knock out of TRIM25 abrogates PAMP-mediated innate immune response; the left panel set shows a western blot where control cells (EV) respond to PAMP but TRM25 KO cells do not respond to PAMP. FIGURE 5B, right panels show graphs depicting the loss of PAMP signaling in TRIM25 KO cells. FIGURE 5C is a western blot that illustrates that TRIM16 has no role in RIG-I -mediated innate immune response induced by constitutively active N-RIG. FIGURE 5D graphically illustrates that TRIM16 has no role in sendai virus-induced innate immune response.

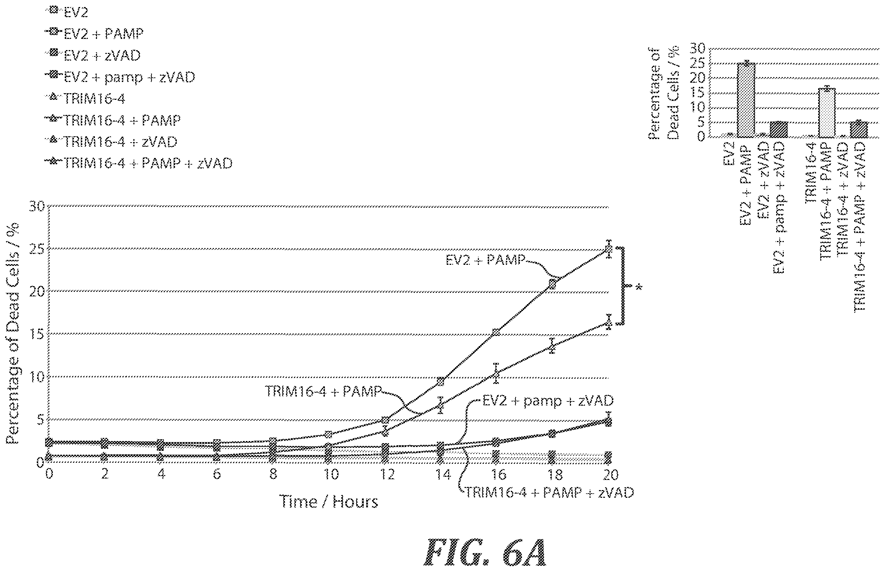

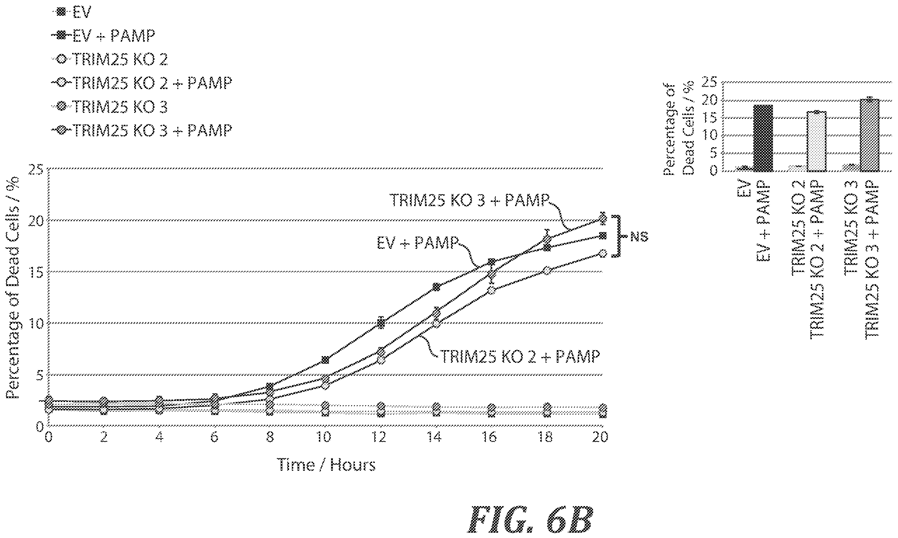

FIGURES 6A and 6B illustrate that TRIM16, but not TRIM25, is involved in RIG-I-mediated induction of apoptosis. FIGURE 6A illustrates that knock out of TRIM16 impairs the induction of apoptosis by HCV PAMP RNA. Compared to EV + PAMP,* P < 0.05. In contrast, FIGURE 6B illustrates that knock out of TRIM25 has no significant effect on the induction of apoptosis.

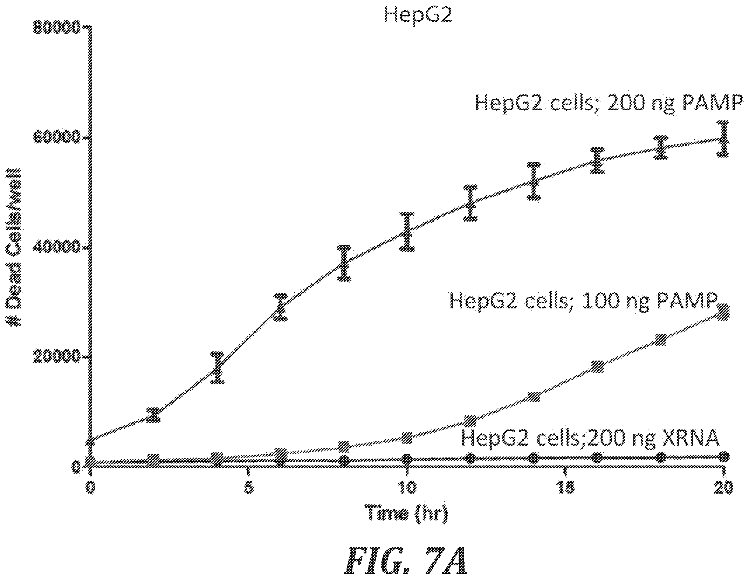

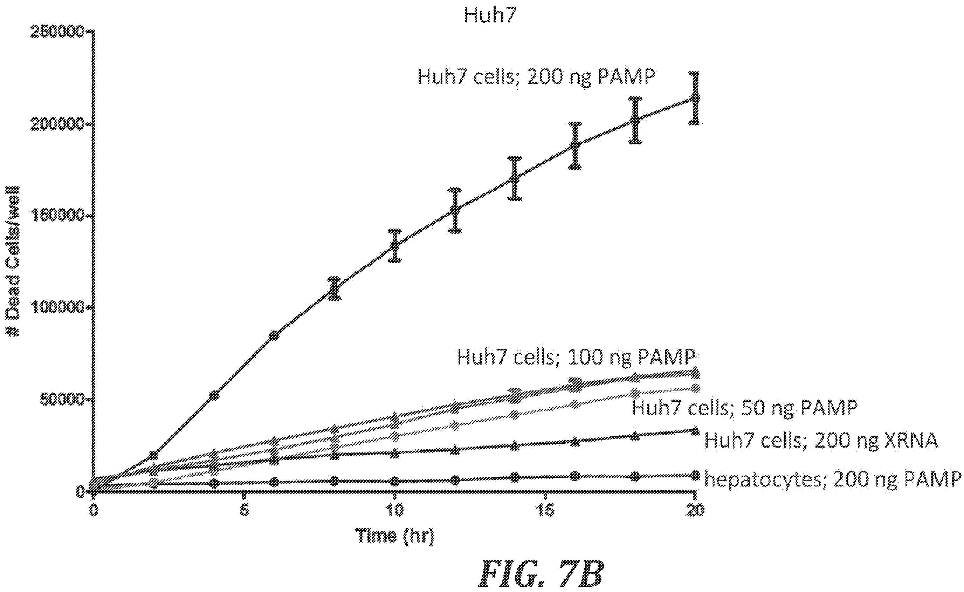

FIGURES 7A and 7B illustrate that PAMP RNA treatment incudes oncolytic destruction of HepG2 (FIGURE 7A) and Huh7 (FIGURE 7B) hepatocellular carcinoma cells but has no effect on primary hepatocytes (FIGURE 7B). Cells were treated with the indicated amount of PAMP RNA or xRNA (negative control) and monitored for Sytox green dye uptake marking permeable/dying or dead cells. Data are plotted as the number of dead cells over a 20 hr time course. PAMP treatment specifically induces the oncolytic destruction of tumor cells (HepG2 and Huh7 cells are human carcinoma-derived cells) but not normal, noncancerous cells (primary human hepatocytes).

### DETAILED DESCRIPTION

The present disclosure is based on research that revealed that inducing RIG-I like receptor (RLR) signaling not only can result in triggering innate immune responses, but can selectively induce apoptosis in cancer cells. This work presents a new therapeutic strategy for combating cancer.

Innate immunity is the body's first line of defense against infection. The innate immune response is also essential for programming effective adaptive immunity and immune memory for a strong and durable immune response critical for eliminating disease-causing pathogens. A key component of the innate immune response against viral infections is the activation of interferon regulatory factor (IRF)3 and the induction of type 1 interferon (IFN). IRF3 induces the expression of antiviral genes and also induces IFN. The antiviral genes can suppress virus replication in the infected cell while IFN directs the suppression of virus replication both in the infected cell and neighboring bystander cells through the expression of hundreds of interferon stimulated genes (ISGs) that have antiviral and immune-modulatory activities. In addition to IFN suppression of viral infections, programed death of virus-infected cells can serve to prevent virus spread. Thus, the combination of IRF3 actions, IFN actions and cell death signaling impart a synergistic program of virus control through yet undefined mechanisms. This disclosure is based on an investigation to determine the interaction between IFN and cell death signaling. These efforts have established that a link between the IFN and cell death responses can be leveraged for therapeutic strategies to control tumor growth.

The induction of IRF activation and IFN is triggered at the onset of virus infection by recognition of pathogen associated molecular patterns (PAMPs) by cellular patter recognition receptors (PRRs), including Toll like receptors (TLR)s, RIG-I like receptors (RLR)s, Nod-like receptors (NLR)s or cyclic GMP-AMP Synthase (cGAS)/Stimulator of Interferon Genes (STING). PRRs detect viral PAMPs or PAMP products to trigger intracellular signaling cascades that ultimately activate transcriptional mechanisms via the interferon regulator factor (IRF)3/7 and Nuclear Factor Kappa B (NF-κB) to direct innate immune activation. In particular RNA viruses are sensed in large part through the actions of RLRs, which are cytosolic RNA helicases that recognize and bind to PAMP RNA motifs. RIG-I is the charter member of the RLR family that also includes MDA5 and LGP2. RIG-I recognizes RNA containing 5' triphosphates (5'ppp) and short dsRNA structure motifs and/or poly-uridine motifs that mark an RNA molecule as non-self. The RIG-I protein structure has a centrally located DExH-box helicase domain, two caspase recruitment domains (CARDs) located at the N-terminus, and a C-terminal repressor domain that regulates signaling by holding the molecule in a closed, signaling-off state until ligand binding leads to its altered conformation of a signaling-on state that drives antiviral defenses. A PAMP motif was previously characterized for RIG-I recognition and binding within the hepatitis C virus (HCV) RNA, which is marked by poly-uridine and single strand RNA structure. When formulated for cell delivery, a synthetic modification of this motif containing 5'ppp (termed PAMP-RNA) is a potent and specific RIG-I agonist that is bound by RIG-I to induce RIG-I signaling. PAMP-RNA directs RIG-I-mediated innate immune actions that provide antiviral and immune-enhancing activity to control virus infection.

Upon recognition and binding of PAMP-RNA, RIG-I undergoes a conformation change that facilitates its interaction with signaling cofactors that confer RIG-I binding to the mitochondria antiviral signaling (MAVS) protein for downstream activation of latent transcription factors, including IRF3, IRF7, and NF-κB transcription factors, causing their translocation to the nucleus and induction of transcriptional programs. The RIG-I signaling pathway is modulated by a variety of RIG-I or MAVS interacting partners including TRAF3, TRAF2, TRAF6, TANK, SIKE, NEMO/IKKγ, FADD, RIP1, HDAC6, TRADD, caspase 8/10, and STING/MITA/MPYS as well as negative signaling regulators such as RNF125, ISG15, DAK, Atg12-Atg5, NLRX1, caspase-1, and several deubiquitinating enzymes, acetylation enzymes, protein kinases, and protein phosphatases. Studies have also shown that RIG-I signaling is also governed through RIG-I ubiquitination or interaction with free ubiquitin, and through reversible phosphorylation and acetylation.

The Tripartite Motif (TRIM) family of proteins is made of over 70 family members with E3 ubiquitin ligase activity and diverse biological functions, including roles in antiviral immunity, autoimmunity, and cancer. TRIM family members have shared conserved sequences that include a gene (RING) domain that recognizes the E2 conjugating enzyme, at least one B-Box domain that mediates self-association in some members, and a coiled-coil (CC) domain that is essential for TRIM dimerization. Members have a variable C-terminal domain that may mediate specific substrate interactions. Many TRIM family members are known to regulate TLR and RLR signaling pathways. Family member TRIM25 regulates RIG-I signaling by K63-polyubiquitination of RIG-I when an exposed RIG-I CARD domain is bound by the C-terminal SPRY domain of TRIM25. The polyubiquitination of RIG-I promotes its interaction with MAVS and subsequent downstream signaling, thus assigning TRIM25 as a critical cofactor of RIG-I innate immune activation. RIG-I interaction with other TRIM family members has not been characterized.

Previous studies have shown that RIG-I activation can induce cell death by various mechanisms. RLR signaling can trigger programmed cell death via induction of Puma and Noxa, Bcl-2 family members that are essential apoptotic regulators. Another series of studies have described the RLR-induced IRF3 mediated Pathway of Apoptosis (RIPA), which requires activation of IRF3 by the RLR signaling pathway, which binds to pro-apoptotic protein BAX and induces it to translocate to the mitochondria and trigger the release of cytochrome C and consequent caspase activation. Interestingly, RIPA is independent of IRF3 transcriptional activity and dependent upon IRF3 ubiquitination following recruitment to the ubiquitinating complex LUBAC by a TRAF2 and TRAF6 dependent mechanism. RIG-I activation can also trigger inflammasome-induced pyroptosis by interaction of the RIG-I CARD domain with the inflammasome components to activate caspase-1 and release of pro-inflammatory cytokines IL-1β and IL-18. Thus, while it is clear that PAMP recognition of RIG-I can trigger programmed cell death, the mechanisms determining whether RIG-I activation leads to innate immune signaling versus cell death are not currently understood.

As described in more detail below, this work addressed the ability of PAMP-RNA to induced cell death within a systematic study of RIG-I-cell death signaling in order to define the molecular determinants responsible for RIG-I-induced cell death. PAMP-RNA dose titration studies defined a threshold dose for triggering RIG-I induced cell death versus innate immune activation. RNA-PAMP induced cell death through RIG-I and caspase-dependent signaling that was enhanced by IFN treatment. Using a proteomics-based screen, a novel RIG-I binding partner, TRIM16, was identified that is essential for PAMP-RNA and RIG-I-induced cell death. Additionally, TRIM16 was shown to bind to the RIG-I CARDs to direct cell-death signaling in response to PAMP-RNA. Thus, TRIM16 is demonstrated to be a cofactor of RIG-I signaling that defines a novel PAMP-sensitive cell death pathway. This is leveraged to demonstrate that RLR signaling is a novel therapeutic target to induce cell death specifically in cancer cells.

In accordance with the foregoing, the invention proposes a composition for use in the treatment of cancer in a subject, as defined in the appended claims.

Thus, the invention proposes a composition for use in the treatment of cancer in a subject, the composition comprising a pathogen-associated molecular pattern (PAMP)-containing nucleic acid molecule, wherein the PAMP-containing nucleic acid molecule comprises:
a. a 5'-arm region comprising a terminal triphosphate,
b. a poly-uracil core comprising at least 8 contiguous uracil residues, and
c. a 3'-arm region comprising at least 8 nucleic acid residues,
wherein the 5'-most nucleic acid residue of the 3'-arm region is not a uracil and wherein the 3'-arm region is at least 30% uracil residues.

In one embodiment of the composition of the invention:
the poly-uracil core consists of between 8 and 30 uracil residues,
the 5'-most nucleic acid residue of the 3'-arm region is a cytosine residue or a guanine residue,
the 3'-arm region is at least 40%, 50%, 60%, 70%, 80%, or 90% uracil residues,
the 3'-arm region comprises at least 7 contiguous uracil residues, and/or
the 5'-arm region further comprises one or more nucleic acid residues disposed between the terminal triphosphate and the poly-uracil core, or the 5'-arm region consists of the terminal triphosphate and wherein the terminal triphosphate is linked directly to the 5'-end of the poly-uracil core,
in any combination.

In another embodiment of the invention, the PAMP-containing nucleic acid molecule induces retinoic acid-inducible gene I (RIG-I) like receptor (RLR) interaction with TRIM16 in a cancer cell in the subject.

Optionally, in this another embodiment, the RLR interaction with TRIM16 induces cell death signaling in the cancer cell, optionally wherein the RLR is RIG-I.

Preferably, the composition of the invention further comprises:
d. interferon (IFN), and/or.
e. a nucleic acid comprising a sequence encoding TRIM16 operatively linked to a promoter sequence, wherein the promoter sequence is capable of inducing expression of the encoded TRIM16 in a cancer cell within the subject, optionally wherein the encoded TRIM16 has an amino acid sequence of at least 90% identity to the amino acid sequence set forth in SEQ ID NO: 1.

Still preferably, the composition of the invention is for use in the treatment of a solid tumor cancer selected from pancreatic cancer, bladder cancer, colorectal cancer, breast cancer, prostate cancer, renal cancer, hepatocellular cancer, lung cancer, ovarian cancer, cervical cancer, gastric cancer, esophageal cancer, head and neck cancer, melanoma, neuroendocrine cancers, CNS cancers, brain tumors, bone cancer, and soft tissue sarcoma, or is a hematological cancer.

Advantageously, the composition of the invention is formulated to be administered in connection with an immunotherapy for the cancer, optionally wherein the immunotherapy comprises cancer- specific antibodies or functional fragments thereof, immune checkpoint inhibitors, and adoptive cell therapies, including CART-cell therapy.

In particular, the composition of the invention is for use in the treatment of cancer in a human subject.

The composition of the invention may also be for use in the treatment of a hematological cancer.

In a preferred embodiment, the composition of the invention further comprises an immunotherapy for the cancer.

Still in a preferred embodiment, the PAMP-containing nucleic acid molecule of the composition of the invention comprises a sequence of SEQ ID NO: 2.

In another preferred embodiment of the invention, the PAMP-containing nucleic acid moleculeof the composition of the invention consists of a sequence of SEQ ID NO: 2.

The present description describes a method of inducing tripartite motif-containing protein 16 (TRIM16) activity in a cell. In some embodiments, the TRIM16 activity is induced by contacting the cell with an effective amount of a retinoic acid-inducible gene I (RIG-I)-like receptor activator. In some embodiments, the RLR activator is a nucleic acid molecule containing a pathogen-associated molecular pattern (PAMP). Thus, in such embodiments, the method comprises contacting the cell with an effective amount of PAMP-containing nucleic acid molecule. The PAMP-containing nucleic acid molecule can also generally be referred to herein as a nucleic acid PAMP. As used herein, a PAMP is a pathogen-associated molecular pattern, i.e., a pattern in the structure/sequence of a nucleic acid molecule, that is recognized by RLR resulting in activation of the RLR in a cell. In some embodiments, the RLR is RIG-I. Exemplary PAMPs and PAMP-containing nucleic acid molecules are disclosed in U.S. Pub. Nos. 2015/0017207 and 2018/0104325, which address PAMP induction of innate immune response signaling. Elements and exemplary embodiments of the PAMP containing nucleic acid encompassed by the disclosure are addressed here.

As a preliminary matter, used herein, the term "nucleic acid" refers to a polymer of monomer units or "residues". The monomer subunits, or residues, of the nucleic acids each contain a nitrogenous base (i.e., nucleobase) a five-carbon sugar, and a phosphate group. The identity of each residue is typically indicated herein with reference to the identity of the nucleobase (or nitrogenous base) structure of each residue. Canonical nucleobases include adenine (A), guanine (G), thymine (T), uracil (U) (in RNA instead of thymine (T) residues) and cytosine (C). However, the nucleic acids of the present disclosure can include any modified nucleobase, nucleobase analogs, and/or non-canonical nucleobase, as are well-known in the art. Modifications to the nucleic acid monomers, or residues, encompass any chemical change in the structure of the nucleic acid monomer, or residue, that results in a noncanonical subunit structure. Such chemical changes can result from, for example, epigenetic modifications (such as to genomic DNA or RNA), or damage resulting from radiation, chemical, or other means. Illustrative and nonlimiting examples of noncanonical subunits, which can result from a modification, include uracil (for DNA), 5-methylcytosine, 5-hydroxymethylcytosine, 5-formethylcytosine, 5-carboxycytosine b-glucosyl-5-hydroxy-methylcytosine, 8-oxoguanine, 2-amino-adenosine, 2-amino-deoxyadenosine, 2-thiothymidine, pyrrolo-pyrimidine, 2-thiocytidine, or an abasic lesion. An abasic lesion is a location along the deoxyribose backbone but lacking a base. Known analogs of natural nucleotides hybridize to nucleic acids in a manner similar to naturally occurring nucleotides, such as peptide nucleic acids (PNAs) and phosphorothioate DNA.

The five-carbon sugar to which the nucleobases are attached can vary depending on the type of nucleic acid. For example, the sugar is deoxyribose in DNA and is ribose in RNA. In some instances herein, the nucleic acid residues can also be referred with respect to the nucleoside structure, such as adenosine, guanosine, 5-methyluridine, uridine, and cytidine. Moreover, alternative nomenclature for the nucleoside also includes indicating a "ribo" or deoxyrobo" prefix before the nucleobase to infer the type of five-carbon sugar. For example, "ribocytosine" as occasionally used herein is equivalent to a cytidine residue because it indicates the presence of a ribose sugar in the RNA molecule at that residue. The nucleic acid polymer can be or comprise a deoxyribonucleotide (DNA) polymer, a ribonucleotide (RNA) polymer, including mRNA. The nucleic acids can also be or comprise a PNA polymer, or a combination of any of the polymer types described herein (e.g., contain residues with different sugars).

In some embodiments, the PAMP-containing nucleic acid is synthetic. In this context, the term "synthetic" refers to non-natural character of the nucleic acid. Such nucleic acids can be synthesized *de novo* using standard synthesis techniques. Alternatively, the nucleic acid PAMPs can be generated or derived from naturally occurring pathogen sequences using recombinant technologies, which are well-known in the art. In some embodiments, the sequence of the synthetic nucleic acid PAMP construct is not naturally occurring.

In some embodiments, the PAMP-containing nucleic acid is an RNA construct. In some of these embodiments, the PAMP-containing nucleic acid is derived from, or reflects the sequence of, the HCV poly-U/UC region and, in this context, may be generally referred to as the poly-U/UC PAMP RNA construct. In some embodiments, the poly-U/UC PAMP RNA construct is synthetic.

The PAMP-containing nucleic acid of this disclosure generally comprises (a) a 5'-arm region comprising a terminal triphosphate; (b) a poly-uracil core (also referred to as a poly-U core); and (c) a 3'-arm region. In one embodiment, the three regions (a, b, and c) are covalently linked in a single nucleic acid polymer macromolecule. The covalent linkage can be direct (without interspersed linker sequence(s)) or indirect (with interspersed linker sequences(s)). In one embodiment, the 5'-arm region is covalently linked to the 5'-end of the poly-U core. In one embodiment, the 3'-arm region is covalently linked to the 3'-arm region of the poly-U core. The polymer can be single or double stranded, or can appear with a combination of single and double stranded portions.

In one embodiment, the poly-U core comprises at least 8 contiguous uracil residues. In further embodiments, the comprises between 8 and 30 contiguous uracil residues, such as 8, 9, 10, 11, 12, 13, 14, 15, 16, 17, 18, 19, 20, 21, 22, 23, 24, 25, 26, 27, 28, 29, or 30 contiguous uracil residues. In one embodiment, the poly-U core comprises more than 8 contiguous uracil residues. In one embodiment, the poly-U core comprises 12 or more contiguous uracil residues. In some embodiments, the poly-U core consists of a plurality of contiguous uracil residues, such as 8, 9, 10, 11, 12, 13, 14, 15, 16, 17, 18, 19, 20, 21, 22, 23, 24, 25, 26, 27, 28, 29, or 30 contiguous uracil residues.

In one embodiment, the 3'-arm region comprises a 5'-most nucleic acid residue that is not a uracil residue. Instead, the 5'-most nucleic acid residue of the 3'-arm region can be an adenine, guanine, or cytosine residue, or any non-canonical residue. In one embodiment, the 5'-most nucleic acid residue of the 3'-arm region is a cytosine residue or a guanine residue.

In one embodiment, the nucleotide composition of the 3'-arm region is at least about 40% uracil residues. In some embodiments, the 3'-arm region is at least about 45%, is at least about 50%, is at least about 60%, is at least about 70%, is at least about 80%, or is at least about 90% uracil residues. In one embodiment, the 3'-arm region comprises a plurality of short stretches (for example, between about 2 and about 15 nucleotides in length) of contiguous uracil residues with one or more cytosine residues interspersed therebetween. In one embodiment, the 3'-arm region comprises a stretch of consecutive uracil residues that does not exceed the length of the poly-U core of the synthetic PAMP-containing nucleic acid molecule. In one embodiment, the 3'-arm region does not comprise a stretch of consecutive uracil residues that exceeds the length of the poly-U core of the synthetic PAMP-containing nucleic acid molecule. In some embodiments, the 3'-arm region comprises at least 7 consecutive uracil residues, such as 7, 8, 9, 10, 11, 12, 13, 14, 15, 16, 17, 18, 19, 20, 21, 22, 23, 24, 25, 26, 27, 28, or 29, contiguous uracil residues.

At a minimum, the 5'-arm region consists of a terminal tri-phosphate (ppp) moiety. In such embodiment, the triphosphate is at the 5'-terminus of the synthetic PAMP-containing nucleic acid molecule and can be represented as "5'-ppp". In a further embodiment, the terminal triphosphate is linked directly to the 5'-end of the poly-U core sequence. In an alternative embodiment, the 5'-arm region comprises the 5'-end terminal triphosphate and one or more additional nucleic acid residues, the sequence of which terminates with a 3'-end. The one or more additional nucleic acid residues in the 5'-arm region of this embodiment are disposed between the terminal triphosphate and the 5'-most uracil residue of the poly-U core. Persons of ordinary skill in the art will readily appreciate that the one or more additional nucleic acid residues in the 5'-arm region can be any number of nucleic acid residues and can present any sequence without limitation. The sequence of the one or more additional nucleic acid residues in the 5'-arm region does not affect the functionality of the PAMP-containing nucleic acid molecule. For instance, as described in U.S. Pub. Nos. 2015/0017207 and 2018/0104325, the addition of a poly-U core region to a non-stimulatory nucleic acid that contains a 5'-triphosphate (such as the HCV X region) confers stimulatory properties for innate immune system signaling. In one embodiment, the sequence of the one or more additional nucleic acid residues in the 5'-arm region does not consist of the entire 5'-end portion of a naturally occurring HCV genome sequence that naturally occurs "upstream" or 5' to the poly-U core of the poly-U/UC region for that HCV strain. Stated differently, in this embodiment the entire synthetic PAMP-containing nucleic acid molecule is not a naturally occurring HCV genome, complete with the 5' triphosphate, the entire coding region, and the untranslated 3' poly-U/UC region. Accordingly, in this embodiment, the 5'-arm region, the one or more nucleic acid residues of the 5'-arm region, and the poly-uracil core do not naturally occur together in an HCV genome. However, in this embodiment, the one or more nucleic acid residues of the 5'-arm region can comprise or consist of a subfragment of the entire naturally occurring sequence that exists between the 5'-arm region and the poly-uracil core. Alternatively, in this embodiment, the one or more nucleic acid residues of the 5'-arm region can comprise sequence in addition to a portion or the entire naturally occurring HCV genome sequence that exists between the 5'-end and the poly-uracil core.

In some embodiments, the PAMP-containing nucleic acid molecule further comprises and additional nucleic acid domain that encodes a functional gene product, such as a polypeptide or interfering RNA construct. The additional nucleic acid domain can be part of the 3'-arm domain, as the whole or part of the one or more additional nucleic acid residues therein. In another embodiment, the additional nucleic acid domain can be disposed between any of the 5'-arm region comprising a terminal triphosphate, the poly-uracil core, and the 3'-arm region.

Non-limiting examples of nucleic acid sequences of PAMPs encompassed by the disclosed PAMP-containing nucleic acids containing are disclosed in U.S. Pub. Nos. 2015/0017207 and 2018/0104325 and are set forth herein as SEQ ID NOS:2-92. The disclosed PAMP-containing nucleic acids can comprise any of the sequences listed therein. It will be understood that such exemplary PAMP containing nucleic acids would comply with the general structural parameters of the PAMP containing molecules, as described herein, including having a terminal tri-phosphate (ppp) moiety. In one embodiment, the PAMP-containing molecule comprises the sequence: GGCCAUCCUGUUUUUUUCCCUUUUUUUUUUUCUCCUUUUUUUUUCCUCUU UUUUUCCUUUUCUUUCCUUU (SEQ ID NO:). In another embodiment, the PAMP-containing nucleic acid comprises the sequence:
GGCCAUUUUCUUUUUUUUUUCUCUUUUUUUUUUUUUUUUUUAUUUUCUUU AAU (SEQ ID NO:). Again, it will be understood that the exemplary PAMP-containing nucleic acid with the indicated sequences will also possess the characteristics described above, including a 5' terminal tri-phosphate (ppp) moiety.

As described in U.S. Pub. Nos. 2015/0017207 and 2018/0104325, nucleic acids with HCV-derived RNA PAMPs having poly-uracil core sequences can trigger retinoic acid-inducible gene I (RIG-I)-like receptor (RLR) signaling. Thus, in some embodiments, the PAMP-containing nucleic acid molecule is capable of inducing retinoic acid-inducible gene I (RIG-I)-like receptor (RLR) activation. In one embodiment, the RLR is RIG-I. Persons of ordinary skill in the art would readily be able to determine the activation of an RLR, such as by assaying the transcription of known downstream RLR-regulated genes, as described in more detail below. For example, in some embodiments, RLR activation can be established by an increase in IFN-β or ISG54 expression. In another embodiment, RLR activation can be established by an increase in IRF-3 phosphorylation.

As described in more detail below, proteomic analyses identified TRIM16 as a key RLR binding partner that specifically leads to cell death signaling. Thus, the PAMP-induced RLR (e.g., RIG-I) signaling includes an RLR-TRIM16 interaction, which can lead to programmed cell death. Accordingly, the method encompasses embodiment wherein the PAMP-containing nucleic acid molecule induces retinoic acid-inducible gene I (RIG-I) like receptor (RLR) interaction with TRIM16 in the cell. An exemplary TRIM16 has an amino acid sequence as set forth in SEQ ID NO: 1, or a functional variant with at least about 80%, about 85%, about 90%, about 95%, about 98%, or about 99% sequence identity thereto. Functional variant indicates that notwithstanding any sequence variation in the protein, the protein retains the activity of the TRIM16. For example, TRIM16, which is also known as EBBP, has been previously characterized identified as an estrogen-responsive gene that has been involved in a number of biological processes, including inhibition of tumor progression in several cancers. The study disclosed herein demonstrates that RLR (e.g., RIG-I)/TRIM16 interaction induces programmed cell death signaling. Accordingly, the TRIM16 activity that is induced can ultimately result in programmed cell death. In some embodiments, the method comprises inducing programmed cell death in the cell. In some embodiments, the PAMP-containing nucleic acid molecule is contacted to the cell at a concentration of about 80ng/mL or greater to results in induction of programmed cell death in the cell.

Accordingly, in some embodiments, the method comprises contacting the cell with the PAMP-containing nucleic acid molecule at a concentration of least about 80ng/mL to about 500ng/mL, such as between 100ng/mL and 250ng/mL. In some embodiments, the method comprises contacting the cell with the PAMP-containing nucleic acid molecule at a concentration of least about 80ng/mL, about 90ng/mL, about 100ng/mL, about 110ng/mL, about 120ng/mL, about 130ng/mL, about 140ng/mL, about 150ng/mL, about 160ng/mL, about 170ng/mL, about 180ng/mL, about 190ng/mL, about 200ng/mL, about 210ng/mL, about 220ng/mL, about 230ng/mL, about 240ng/mL, about 250ng/mL, about 260ng/mL, about 270ng/mL, about 280ng/mL, about 290ng/mL, about 300ng/mL, about 310ng/mL, about 320ng/mL, about 330ng/mL, about 340ng/mL, about 350ng/mL, about 360ng/mL, about 370ng/mL, about 380ng/mL, about 390ng/mL, about 400ng/mL, about 410ng/mL, about 420ng/mL, about 430ng/mL, about 440ng/mL, about 450ng/mL, about 460ng/mL, about 470ng/mL, about 480ng/mL, about 490ng/mL, and about 500ng/mL.

In some embodiments, the method further comprises contacting the cell with interferon (IFN). In some embodiments, the IFN is type 1 IFN. In some embodiments, the IFN is pegylated IFN. In some embodiments, the IFN is IFN lambda.

In some embodiments, the cell is contacted with about 5 to about 1500 units IFN per ml, such as about 100 to about 1000 units IFN per ml, such as about 100 to about 500 units IFN per ml, such as about 200 to about 1000 units IFN per ml, such as about 300 to about 700 units IFN per ml, and such as about 500 to about 1000 units IFN per ml. Exemplary doses of IFN include about 100 units IFN per ml, about 200 units IFN per ml, about 300 units IFN per ml, about 400 units IFN per ml, about 500 units IFN per ml, about 600 units IFN per ml, about 700 units IFN per ml, about 800 units IFN per ml, about 900 units IFN per ml, about 1000 units IFN per ml, about 1200 units IFN per ml, and about 1500 units IFN per ml. Use herein of the term "units" refers to International units.

The TRIM16 activity can be facilitated or further enhanced by also providing the cell with a nucleic acid encoding TRIM16. As indicated above, an exemplary TRIM16 protein sequence is set forth in SEQ ID NO:1 or a functional variant with at least about 80%, about 85%, about 90%, about 95%, about 98%, or about 99% sequence identity thereto. Thus, the nucleic acid can be any nucleic acid that encodes an amino acid sequence as set forth in SEQ ID NO:1 or a functional variant with at least about 80%, about 85%, about 90%, about 95%, about 98%, or about 99% sequence identity thereto. The nucleic acid can be operatively linked to a promoter sequence, wherein the promoter sequence is capable of inducing expression of the encoded TRIM16 in the cell. The term "promoter" refers to a regulatory nucleotide sequence that can activate transcription (expression) of a gene and/or splice variant isoforms thereof. A promoter is typically located upstream of a gene, but can be located at other regions proximal to the gene, or even within the gene. The promoter typically contains binding sites for RNA polymerase and one or more transcription factors, which participate in the assembly of the transcriptional complex. As used herein, the term "operatively linked" indicates that the promoter and the encoding nucleic acid are configured and positioned relative to each other a manner such that the promoter can activate transcription of the encoding nucleic acid by the transcriptional machinery of the cell. The promoter can be constitutive or inducible. Constitutive promoters can be determined based on the character of the target cell and the particular transcription factors available in the cytosol. A person of ordinary skill in the art can select an appropriate promoter based on the intended person, as various promoters are known and commonly used in the art

The cell can be any cell for which TRIM16 activity is desired. In some embodiments, the cell is a cancer cell. The cancer cell can be from a solid tumor or a hematological cancer.

Representative solid tumors are pancreatic cancer, bladder cancer, colorectal cancer, breast cancer, prostate cancer, renal (kidney) cancer, hepatocellular cancer, lung cancer, ovarian cancer, cervical cancer, gastric cancer, esophageal cancer, head and neck cancer, melanoma, neuroendocrine cancers, CNS cancers, brain tumors, bone cancer, soft tissue sarcoma. Representative hematological cancers include leukemias, lymphomas, and myelomas.

In some embodiments, the method can be performed such that the cell is contacted with the effective amount of the PAMP-containing nucleic acid molecule *in vitro.* Typically, one or more cells are maintained in a suitable culture medium. The amount of the PAMP-containing nucleic acid molecule is administered to the cells in the culture medium at the desired concentration in a carrier that is amenable to continued culture of the cells.

The method described here can be performed *in vivo,* such as in a human or non-human mammalian subject (such as another primate, horse, dog, cat, mouse, rat, guinea pig, rabbit, and the like) with a disease or condition that would benefit from induction of TRIM16 activity. Considering that activation of TRIM16 via sufficient PAMP signaling of RLR (e.g., RIG-I) leads to apoptosis, the method is advantageously performed with the disease or condition in the subject being a cancer, such as solid tumor cancer or hematological cancer. Exemplary cancers encompassed by the disclosure include those described above including pancreatic cancer, bladder cancer, colorectal cancer, breast cancer, prostate cancer, renal (kidney) cancer, hepatocellular cancer, lung cancer, ovarian cancer, cervical cancer, gastric cancer, esophageal cancer, head and neck cancer, melanoma, neuroendocrine cancers, CNS cancers, brain tumors, bone cancer, soft tissue sarcoma, and myelomas. In these *in vivo* embodiments, the effective amount of the PAMP-containing nucleic acid is administered to the subject in a manner appropriate for the particular disease (e.g., cancer). In some embodiments, the method is an aspect of a combination therapy and thus further comprises administering to the subject an immunotherapy, such as cancer-specific antibodies or functional fragments thereof, immune checkpoint inhibitors, and adoptive cell therapies, including CAR T-cell therapy.

Thus, in another aspect, the description describes a method of treating cancer in a subject in need thereof. The method comprises administering to the subject a therapeutically effective amount of a pathogen-associated molecular pattern (PAMP)-containing nucleic acid molecule.

As used herein, the term "treat" refers to medical management of a disease, disorder, or condition (e.g., cancer, as described above) of a subject (e.g., a human or non-human mammal, such as another primate, horse, dog, mouse, rat, guinea pig, rabbit, and the like). Treatment can encompasses any indicia of success in the treatment or amelioration of a disease or condition (e.g., a cancer), including any parameter such as abatement, remission, diminishing of symptoms or making the disease or condition more tolerable to the subject, slowing in the rate of degeneration or decline, or making the degeneration less debilitating. Specifically in the context of cancer, the term treat can encompass slowing or inhibiting the rate of cancer growth, or reducing the likelihood of recurrence, compared to not having the treatment. In some embodiments, the treatment encompasses resulting in some detectable degree of cancer cell death in the patient. The treatment or amelioration of symptoms can be based on objective or subjective parameters, including the results of an examination by a physician. Accordingly, the term "treating" includes the administration of the compositions of the present disclosure to alleviate, or to arrest or inhibit development of the symptoms or conditions associated with disease or condition (e.g., cancer). The term "therapeutic effect" refers to the amelioration, reduction, or elimination of the disease or condition, symptoms of the disease or condition, or side effects of the disease or condition in the subject. The term "therapeutically effective" refers to an amount of the composition that results in a therapeutic effect and can be readily determined.

In some embodiments, the PAMP-containing nucleic acid molecule comprises a 5'-arm region comprising a terminal triphosphate, a poly-uracil core comprising at least 8 contiguous uracil residues, and a 3'-arm region comprising at least 8 nucleic acid residues, wherein the 5'-most nucleic acid residue of the 3'-arm region is not a uracil and wherein the 3'-arm region is at least 30% uracil residues. A detailed description of the PAMP-containing nucleic acid molecule is provided above is not repeated here for brevity. As described, PAMP-containing molecule is functional to induce induces retinoic acid-inducible gene I (RIG-I) like receptor (RLR) interaction with TRIM 16 in a cancer cell in the subject. In some embodiments, the RLR is RIG-I. The RLR interaction with TRIM16 results in induction of cell death signaling in a cancer cell in the subject.

It has been demonstrated that the interferon (IFN) can interact synergistically with programmed cell death signaling to result in enhanced destruction of cells and even tumors. Indeed, the study described below establishes that the PAMP-induced cell death (via stimulation of the RLR/TRIM16 interaction) induces caspase-dependent cell death that is further enhanced by the addition of IFN treatment. Accordingly, in some embodiments the method of treatment further comprises administering to the subject an effective amount of interferon (IFN) to the subject. In some embodiments, the IFN is type 1 IFN. In some embodiments, the IFN is pegylated IFN. In some embodiments, the IFN is IFN lambda.

IFN can be dosed at any therapeutically effective amount as determined by persons of ordinary skill in the art. Exemplary doses ranges include about 5 to about 1500 units IFN per ml, such as about 100 to about 1000 units IFN per ml, such as about 100 to about 500 units IFN per ml, such as about 200 to about 1000 units IFN per ml, such as about 300 to about 700 units IFN per ml, and such as about 500 to about 1000 units IFN per ml. Exemplary doses of IFN include about 100 units IFN per ml, about 150 units IFN per ml, about 200 units IFN per ml, about 250 units IFN per ml, about 300 units IFN per ml, about 350 units IFN per ml, about 400 units IFN per ml, about 450 units IFN per ml, about 500 units IFN per ml, about 550 units IFN per ml, about 600 units IFN per ml, about 650 units IFN per ml, about 700 units IFN per ml, about 750 units IFN per ml, about 800 units IFN per ml, about 850 units IFN per ml, about 900 units IFN per ml, about 950 units IFN per ml, about 1000 units IFN per ml, about 1200 units IFN per ml, and about 1500 units IFN per ml. Use herein of the term "units" refers to International units.

In some embodiments, the method further comprises administering to the subject a nucleic acid comprising a sequence encoding TRIM16 operatively linked to a promoter sequence. As described above, the nucleic acid can encode a TRIM16 protein comprising an amino acid sequence as set forth in SEQ ID NO:1, or a functional variant with at least about 80%, about 85%, about 90%, about 95%, about 98%, or about 99% sequence identity thereto. The promoter can be selected such that it is operative to induce expression of the TRIM in the cancer cell. Such promoter can be configured for inducible or constitutive expression in the intended target cancer cell type according to conventional knowledge in the art. The nucleic acid (and the promoter) can be incorporated into an appropriate vector to facilitate delivery and expression of the encoding nucleic acid in the cancer cell. The vector can be a viral vector, circular nucleic acid construct (e.g., plasmid), or nanoparticle. Various viral vectors are known in the art and are encompassed by the present disclosure. See, e.g., Machida, C. A. (ed.), Viral Vectors for Gene Therapy: Methods and Protocols, Humana Press, Totowa, New Jersey (2003); Muzyczka, N., (ed.), Current Topics in Microbiology and Immunology: Viral Expression Vectors, Springer-Verlag, Berlin, Germany (2012). In some embodiments, the viral vector is an adeno associated virus (AAV) vector, an adenovirus vector, a retrovirus vector, or a lentivirus vector. A specific embodiment of an AAV vector includes the AAV2.5 serotype.

The method can be useful to treat any form of cancer (e.g., solid tumor or hematological cancer) as long as the cancerous cells have maintained the requisite TRIM16 signaling pathway. Exemplary cancers encompassed by this aspect are described above, and include but are not limited to pancreatic cancer, bladder cancer, colorectal cancer, breast cancer, prostate cancer, renal (kidney) cancer, hepatocellular cancer, lung cancer, ovarian cancer, cervical cancer, gastric cancer, esophageal cancer, head and neck cancer, melanoma, neuroendocrine cancers, CNS cancers, brain tumors, bone cancer, soft tissue sarcoma, and myelomas.

Considering that a functional effect of the PAMP-containing nucleic acid is induction of programmed cell death signaling in the cell, the method of this aspect is particularly applicable to combination with other anti-cancer therapies. Accordingly, in some embodiments the method further comprises administering to the subject an anti-cancer therapeutic. The anti-cancer therapeutic can be any toxin, small molecule, large molecule therapeutic that has demonstrable therapeutic effect on cancer cells in a subject. In some embodiments, the method further comprises administering to the subject an immunotherapy for the cancer, such as cancer-specific antibodies or functional fragments thereof, immune checkpoint inhibitors, and adoptive cell therapies, including CAR T-cell therapy. The disclosed PAMP-containing nucleic acid can be administered concurrently with or separate from the additional anti-cancer therapeutics, including IFN and/or TRIM16 encoding nucleic acids.

In another aspect, the invention provides a therapeutic composition comprising a pathogen-associated molecular pattern (PAMP)-containing nucleic acid molecule and an additional therapeutic agent. In some embodiments, the PAMP-containing nucleic acid molecule comprises a 5'-arm region comprising a terminal triphosphate, a poly-uracil core comprising at least 8 contiguous uracil residues, and a 3'-arm region comprising at least 8 nucleic acid residues, wherein the 5'-most nucleic acid residue of the 3'-arm region is not a uracil and wherein the 3'-arm region is at least 30% uracil residues. A more detailed description of the PAMP-containing nucleic acid molecule is provided above is not repeated here for brevity. As described, PAMP-containing molecule is functional to induce induces retinoic acid-inducible gene I (RIG-I) like receptor (RLR) interaction with TRIM16 in a cancer cell in the subject. In some embodiments, the RLR is RIG-I. The RLR interaction with TRIM16 results in induction of cell death signaling in a cancer cell in the subject

In one embodiment, the additional therapeutic agent can be interferon (IFN), as described above in more detail. In some embodiments, the IFN is type 1 IFN. In some embodiments, the IFN is pegylated IFN. In some embodiments, the IFN is IFN lambda. In one embodiment, the additional therapeutic agent is a nucleic acid encoding comprising a sequence encoding TRIM16 operatively linked to a promoter sequence. As described above, the nucleic acid can encode a TRIM16 protein comprising an amino acid sequence as set forth in SEQ ID NO:1, or a functional variant with at least about 80%, about 85%, about 90%, about 95%, about 98%, or about 99% sequence identity thereto. The promoter can be selected such that it is operative to induce expression of the TRIM in the cancer cell. In another embodiment, the therapeutic composition comprises the PAMP-containing nucleic acid, IFN, and a nucleic acid encoding TRIM16 operatively linked to a promoter sequence.

The invention encompasses formulations of the therapeutic composition configured in a manner appropriate for methods of administration for application to *in vivo* therapeutic settings in subjects (e.g., mammalian subjects with cancer). Administration can be by any procedure known in the art, including but not limited to, oral, parenteral, intraspinal, intracisternal, subdural, rectal, intradermal, transdermal, intramuscular, or topical administration. In a specific embodiment, the administration is intratumoral. To facilitate delivery, the therapeutic composition can be formulated in various compositions with a pharmaceutically acceptable carrier, excipient or diluent. "Pharmaceutically acceptable" means the carrier, excipient or diluent of choice does not adversely affect the biological activity of the PAMP-containing nucleic acid molecule and any additional therapeutic agent, or the recipient of the composition.

According to skill and knowledge common in the art, the disclosed PAMP-containing nucleic acid and additional therapeutic agent, including IFN, encoding nucleic acids and/or vectors comprising the nucleic acids, toxins, immunotherapy compounds, etc., can be formulated in any appropriate therapeutically delivery system or form. Exemplary, non-limiting delivery systems can include particle formulations, such as emulsions, microparticles, and nanoparticles, which can be, for example, particles and/or matrices, and liposomes, and the like, which are advantageous for the delivery of antigens. In one embodiment, the disclosed PAMP-containing nucleic acid and additional therapeutic agent are formulated into a liposomal delivery format. Exemplary applications of liposomal formulations are described in Yallapu, U., et al., Liposomal Formulations in Clinical Use: An Updated Review, Pharmaceutics 9(2):12 (2017).

### General definitions

Unless specifically defined herein, all terms used herein have the same meaning as they would to one skilled in the art of the present disclosure. Practitioners are particularly directed to Ausubel, F.M., et al. (eds.), Current Protocols in Molecular Biology, John Wiley & Sons, New York (2010), Coligan, J.E., et al. (eds.), Current Protocols in Immunology, John Wiley & Sons, New York (2010), Mirzaei, H. and Carrasco, M. (eds.), Modern Proteomics - Sample Preparation, Analysis and Practical Applications in Advances in Experimental Medicine and Biology, Springer International Publishing, 2016, and Comai, L, et al., (eds.), Proteomic: Methods and Protocols in Methods in Molecular Biology, Springer International Publishing, 2017, for definitions and terms of art.

For convenience, certain terms employed herein, in the specification, examples and appended claims are provided here. The definitions are provided to aid in describing particular embodiments and are not intended to limit the claimed invention, because the scope of the invention is limited only by the claims.

The use of the term "or" in the claims is used to mean "and/or" unless explicitly indicated to refer to alternatives only or the alternatives are mutually exclusive, although the disclosure supports a definition that refers to only alternatives and "and/or."

The words "a" and "an," when used in conjunction with the word "comprising" in the claims or specification, denotes one or more, unless specifically noted.

Unless the context clearly requires otherwise, throughout the description and the claims, the words "comprise," "comprising," and the like, are to be construed in an inclusive sense as opposed to an exclusive or exhaustive sense, which is to indicate, in the sense of "including, but not limited to." Words using the singular or plural number also include the plural and singular number, respectively. The word "about" indicates a number within range of minor variation above or below the stated reference number. For example, "about" can refer to a number within a range of 10%, 9%, 8%, 7%, 6%, 5%, 4%, 3%, 2%, or 1% above and/or below the indicated reference number.

As used herein, the term "polypeptide" or "protein" refers to a polymer in which the monomers are amino acid residues that are joined together through amide bonds. When the amino acids are alpha-amino acids, either the L-optical isomer or the D-optical isomer can be used, the L-isomers being preferred. The term polypeptide or protein as used herein encompasses any amino acid sequence and includes modified sequences such as glycoproteins. The term polypeptide is specifically intended to cover naturally occurring proteins, as well as those that are recombinantly or synthetically produced.

One of skill will recognize that individual substitutions, deletions or additions to a peptide, polypeptide, or protein sequence which alters, adds or deletes a single amino acid or a percentage of amino acids in the sequence is a "conservatively modified variant" where the alteration results in the substitution of an amino acid with a chemically similar amino acid. Conservative amino acid substitution tables providing functionally similar amino acids are well known to one of ordinary skill in the art. The following six groups are examples of amino acids that are considered to be conservative substitutions for one another:
(1) Alanine (A), Serine (S), Threonine (T),
(2) Aspartic acid (D), Glutamic acid (E),
(3) Asparagine (N), Glutamine (Q),
(4) Arginine (R), Lysine (K),
(5) Isoleucine (I), Leucine (L), Methionine (M), Valine (V), and
(6) Phenylalanine (F), Tyrosine (Y), Tryptophan (W).

Reference to sequence identity addresses the degree of similarity of two polymeric sequences, such as protein sequences. Determination of sequence identity can be readily accomplished by persons of ordinary skill in the art using accepted algorithms and/or techniques. Sequence identity is typically determined by comparing two optimally aligned sequences over a comparison window, where the portion of the peptide or polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical amino-acid residue or nucleic acid base occurs in both sequences to yield the number of matched positions, dividing the number of matched positions by the total number of positions in the window of comparison and multiplying the result by 100 to yield the percentage of sequence identity. Various software driven algorithms are readily available, such as BLAST N or BLAST P to perform such comparisons.

Disclosed are materials, compositions, and components that can be used for, can be used in conjunction with, can be used in preparation for, or are products of the disclosed methods and compositions. It is understood that, when combinations, subsets, interactions, groups, etc., of these materials are disclosed, each of various individual and collective combinations is specifically contemplated, even though specific reference to each and every single combination and permutation of these compounds may not be explicitly disclosed. This concept applies to all aspects of this disclosure including, but not limited to, steps in the described methods. Thus, specific elements of any foregoing embodiments can be combined or substituted for elements in other embodiments. For example, if there are a variety of additional steps that can be performed, it is understood that each of these additional steps can be performed with any specific method steps or combination of method steps of the disclosed methods, and that each such combination or subset of combinations is specifically contemplated and should be considered disclosed. Additionally, it is understood that the embodiments described herein can be implemented using any suitable material such as those described elsewhere herein or as known in the art.

Publications cited herein and the subject matter for which they are cited are hereby specifically incorporated by reference in their entireties.

The following describes a study demonstrating that induction of RLR signaling using PAMP-RNA can induce programmed cell death and can be applied in a cancer-specific context.

### Introduction

Retinoic acid-inducible gene I (RIG-I) functions as a cytosolic pathogen recognition receptor by binding the pathogen associated molecular patterns (PAMPs) in viral RNA. The molecular signature for RIG-I recognition of viral RNA has been identified as specific motifs containing 5'-triphosphate (5'ppp), including poly-uridine (poly-U)-rich RNA motifs and short double stranded (ds) RNA motifs that are found within viral RNA products that accumulate in the cytoplasm of infected cells during RNA virus replication. The inventor previously developed a synthetic 5'ppp RNA RIG-I ligand based on the poly-U motif of the hepatitis C virus (HCV) RNA (PAMP-RNA) that is specifically recognized and bound by RIG-I. PAMP-RNA activation of RIG-I triggers intracellular signaling cascades that mediate RIG-I effector functions to induce innate immunity and provide antiviral therapeutic and immune adjuvant actions.

This study demonstrates the surprising finding that PAMP-RNA activation of RIG-I can induce apoptotic cell death. Dose range studies of PAMP-RNA actions revealed that low dose PAMP-RNA levels trigger innate immune activation by RIG-I while increased PAMP-RNA levels mediated RIG-I signaling of cell death. This reveals a novel cell death pathway of PAMP-RNA sensitivity and actions. Proteomic analyses identified TRIM16 as a key RIG-I binding partner that specifically directs cell death induced by PAMP-RNA through RIG-I signaling. TRIM16 was necessary for cell death signaling but not innate immune activation by RIG-I. Additionally, TRIM16-mediated cell death was further enhanced by type I interferon (IFN). These studies demonstrate that TRIM16 is a RIG-I binding partner that directs cell death in response to PAMP-RNA treatment. PAMP-RNA signaling and the TRIM16/RIG-VIFN axis is, thus, demonstrated as a novel tumor suppressor program of cell death for applications in cancer therapy.

### Results

### HCV PAMP RNA-induced apoptosis is dose-dependent

Studies have found that RIG-I agonists can induce both innate immune signaling activation and apoptosis. To confirm this with the PAMP-RNA, a dose-titration study was performed to evaluate the amount of PAMP-RNA required to induce each biological event in Huh7 cells (FIGURES 1A-1D). Interestingly, innate immune signaling was induced at a threshold of 10ng/mL and greater as evidenced by induction of IRF3 induced expression of ISG56 and ISG15 and IFN-dependent IFITM1, while IncuCyte^{®} analysis of PAMP-RNA-induced cell death occurred at a minimal concentration of 100ng/mL. These titration studies indicate that the PAMP-RNA induced the highest level of cell death at 200 ng/mL (FIGURES 1C and 1D). Thus, 200 ng/mL was used as the optimal concentration for inducing RIG-I-mediated apoptosis and for PAMP-RNA induction of cell death for the remainder of the studies.

### PAMP-RNA induction of apoptosis is RIG-I and caspase dependent

To confirm that the PAMP-RNA drives RIG-I-dependent signaling, and to assess the role of caspase activation in the cell death response to RIG-I signaling _ENREF_27induced by PAMP-RNA, the extent of cell death and caspase activity induced by HCV PAMP was evaluated in RIG-I knockdown cells and empty vector control cells with or without treatment with the pan-caspase inhibitor zVAD. Indeed, loss of RIG-I in the RIG-I knock out cells abrogated PAMP-RNA induced cell death, while zVAD treatment of empty vector control cells prior to PAMP-RNA treatment, reduced the frequency of induced cell death (FIGURE 2A-1,-2). Immunoblot analysis of innate immune signaling protein expression showed that zVAD treatment did not impact PAMP-RNA induction of ISG56, indicating that inhibition of caspases did not inhibit innate immune responses triggered by PAMP-RNA in these cells (FIGURE 2B). Furthermore, loss of RIG-I in the cells decreased the expression of p53-dependent pro-apoptotic genes NOXA and PUMA (FIGURE 3C), which are known to be induced during RIG-I dependent apoptosis. Thus, PAMP-RNA can drive cell death through RIG-I and caspase-dependent processes.

### Type I IFN pathway is involved in PAMP-RNA-mediated cell death

To determine the impact of IFN on PAMP-RNA signaling of cell death, cell death was evaluated in Huh7 control cells and cells lacking IFNAR or RIG-I. Loss of IFNAR was shown to partially abrogate RIG-I mediated induction of cell death triggered by PAMP-RNA (FIGURE 3A). This finding was further confirmed with the partial impairment of RIG-I mediated induction of apoptosis in Huh7 cells pre-treated with an inhibitor of type I IFN signaling, B 18R (FIGURE 3B).

### TRIM16 is a novel RIG-I binding factor

To identify possible cofactors of RIG-I involved in cell innate immune or cell death signaling by PAMP-RNA, a proteomics screen of P85CH8 cells was performed for RIG-I binding proteins after RIG-I activation by Sendai virus (SeV) (FIGURE 4A). SeV is a potent RIG-I activator and was therefore used in these studies as a PAMP-RNA surrogate to provide strong and uniform activation of RIG-I in the cell cultures. Cells were transfected with a FLAG-tagged RIG-I construct or FLAG construct control, and either mock infected or infected with SeV. 16 hrs later RIG-I and RIG-I-associated proteins were isolated by FLAG-affinity purification and identified by LC/MS-MS. The screen identified known RIG-I interactors of innate immunity, such as OASL. Importantly, the analyses identified 5 unique peptide sequences associated with FLAG-RIG-I but not FLAG beads alone corresponding to TRIM16, an E3 ubiquitin ligase. TRIM16 is not previously known to interact with RIG-I. The status of TRIM16 and RIG-I was binding partners was confirmed using co-immunoprecipitation in HEK293 cells expressing RIG-I-FLAG and TRIM16 V5 constructs, (FIGURE 4B). The domains essential for the RIG-I and TRIM16 interaction were further mapped by co-immunoprecipitation analysis of co-expressed truncation mutants from each protein. These analyses revealed that the RIG-I CARD domains (aa 1-176) are necessary and sufficient for RIG-I association with TRIM16 (FIGURE 4C). Likewise, the reciprocal co-immunoprecipation with TRIM16 truncations revealed that the B-Box domains of TRIM16 are necessary and sufficient for the TRIM16 association with RIG-I (FIGURE 4D).

### Loss of TRIM16 does not impact RIG-I induction of innate immune responses

To elucidate a possible role of TRIM16 in innate immune signaling, the impact of TRIM16 loss on PAMP-RNA-induced innate immune activation was evaluated. TRIM16 expression was depleted in Huh7 cells using CRISPR/cas9 targeted knockout of TRIM16. In contrast to Huh7 cell lines engineered to knockout expression of RIG-I or TRIM25 (a positive regulator of RIG-I signaling), the Huh7 TRIM16 KO cell lines from two different CRISPR/cas9 targeting sequences both exhibited robust induction of the RIG-I innate immune signaling by PAMP-RNA as evidenced by induction of RIG-I-responsive genes ISG56, ISG15, IFNβ, and TNFα (FIGURES 5A and 5B). Moreover, loss of TRIM16 expression did not impact RIG-I downstream innate signaling programs in the presence of a dominant active form of RIG-I (N-RIG) or after SeV infection (FIGURES 5C and 5D). Together, these experiments demonstrate that TRIM16 is not involved with the RIG-I-induced host innate immune signaling processes.

### TRIM16, but not TRIM25, is involved in RIG-I-mediated apoptosis

To determine if TRIM16 is specifically involved in RIG-I-mediated cell death induced by PAMP-RNA, the impact of TRIM16 loss on PAMP-RNA-induced cell death was evaluated. TRIM16 KO and TRIM25 KO Huh7 cells were treated with either PAMP-RNA, caspase inhibitor (ZVAD) or both, and were evaluated by real-time imaging cell death assay. Loss of TRIM16 or ZVAD treatment inhibited PAMP-RNA-induced cell death compared to the controls (FIGURE 6A). In contrast, loss of TRIM25 expression did not impact the extent of PAMP-RNA-induced cell death (FIGURE 6B). These studies reveal that PAMP-RNA specifically signals RIG-I-mediated apoptotic cell death through the novel RIG-I binding partner TRIM16.

### PAMP-RNA-mediated cell death is specific for cancer cells

To determine if RLR (e.g., RIG-I) mediated programmed cell death signaling is equally impactful on healthy and cancer cells, PAMP RNA was exposed to both hepatic cancer cells lines and normal primary human hepatocytes. Specifically, HEPG2 and Huh7 hepatocellular carcinoma cells and primary hepatocytes were treated with increasing amounts of PAMP RNA or xRNA (use as a negative control) and monitored for over a course of at least 24 hours for Sytox green dye uptake marking permeable/dying or dead cells. The PAMP treatment was observed to specifically induce the oncolytic destruction of tumor cells (i.e., the HepG2 and Huh7 cells, which are human carcinoma-derived cells) but not normal, noncancerous primary human hepatocytes. Specifically, FIGURES 7A and 7B illustrate that PAMP RNA treatment incudes oncolytic destruction of HepG2 (FIGURE 7A) and Huh7 (FIGURE 7B) hepatocellular carcinoma cells but has no effect on primary hepatocytes (FIGURE 7B).

This demonstrates that healthy cells are refractory to PAMP-induced programmed cell death signaling through RLR (e.g., RIG-I). Accordingly, cancer cells can be specifically targeted for destruction while minimizing or preventing off-site toxicity in healthy cells.

### Discussion

This study demonstrates that TRIM16 is an effector of cell death signaling in the PAMP-RNA/RIG-I pathway. These data support a model of TRIM16 actions in which PAMP-RNA, and other RIG-I ligands, activate RIG-I to confer binding to TRIM16. As a result, TRIM16 directs the downstream activation of caspases to mediate apoptotic cell death. That induction of RIG-I-mediated cell death occurs at higher dose of PAMP-RNA than does RIG-I signaling of innate immune activation likely underscores differential actions of innate immunity to protect the cell from infection versus apoptosis to delete the infected cells for protection of the tissue and organism. Thus, higher dose PAMP-RNA treatment of cells and tissue presents an approach for strategies for directed cell death outcome wherein TRIM16 mediates a cell death axis of PAMP-RNA/RIG-I signaling.

TRIM16 (also known as EBBP) was earlier identified as an estrogen-responsive gene that has been involved in a number of biological processes. TRIM16 has been mapped to the regulation of stress-induced protein aggregates and regulating autophagy in endomembrane damage homeostasis. TRIM16 has been linked to keratinocyte differentiation and retinoid metabolism. One study has found a correlation between TRIM16 expression and hyperplasia of the synovium in rheumatoid arthritis. Another study found that TRIM16 interacts with inflammasome components caspase 1 and NLRP1 to enhance IL-1b secretion, suggesting that TRIM16 plays a role in inflammasome regulation. However, most notably, TRIM16 has been prominently linked to tumor suppression in cancer. Several mechanisms of TRIM16-mediated tumor suppression have been elucidated. Studies in lung, ovarian, and breast cancer cell model systems have found that TRIM16 inhibits the epithelial to mesenchymal transition (EMT) during tumor progression by downregulating the tumor promoting sonic hedgehog pathway. Likewise, cellular models of hepatocellular carcinoma and prostate cancer have shown TRIM16 inhibits EMT by suppression of transcriptional inhibitors that regulate expression of the cell-cell adhesion molecule E-cadherin. A study of TRIM16 in melanoma found that TRIM16 inhibits proliferation and migration by regulating IFN-beta 1 production. Studies have also found that TRIM16 regulates the stability of the intermediate filament family member vimentin in tumors, reduces skin cancer cell migration, and has been shown to inhibit neuroblastoma growth by inhibiting the cell cycle. Finally, studies in breast cancer cell lines have found that overexpression of TRIM16 induced apoptosis through activation of caspase-2.

Of note is that the FishTRIM16L gene that was cloned from the orange spotted grouper and has 29% identity to *homo sapiens,* and was shown to antagonize innate immune signaling in fish cells. In contrast, this study found that loss of humanTRIM16 did not impact PAMP-RNA/RIG-induction of innate immune activation. Likely this difference in function between fish and human TRIM16 in innate immune response is due to lack of sequence identity, whereas the fish TRIM16L protein lacks the B-box domain and is not expected to bind to RIG-I but likely imparts distinct actions on fish innate immunity.

Interestingly, the present studies revealed that HCV PAMP-induced cell death was partially dependent on IFN, which is in direct contrast to a previous study that showed RIPA is independent of IFN. Without being bound by any particular theory, this apparent discrepancy is likely due to the use of different cell lines in the two studies but serves to show that IFN can enhance cell death signaling, consistent with IFN proapoptotic actions. As RIG-I itself is an ISG, IFN can serve to increase RIG-I levels to enhance PAMPR-RNA signaling strength for downstream engagement of TRIM16. Alternatively, other ISGs could cooperate with PMPA-RNA signaling to enhance the TRIM16 cell death phenotype.

In summary, this study identified TRIM16 as a novel RIG-I binding partner that mediates cell death signaling induced by PAMP-RNA. The effect was observed to be specific for cancer cells and did not negatively affect healthy cells of the same originating tissue. Thus, targeting this novel RIG-I/TRIM16 pathway with PAMP-RNA to direct cell death outcome is a viable strategy for therapeutic approaches aimed at directing cell death, in cancer cells.

The following examples are provided for the purpose of illustrating, not limiting, the disclosure.

### Example 1

### Exemplary methods and materials

### Cell culture, inhibitors, and virus

Human Huh 7 hepatocellular carcinoma, primary immortalized human PH5CH8 hepatocytes, and human embryonic kidney HEK293 cells were grown in "complete DMEM" (Dulbecco's modified Eagle medium (DMEM) supplemented with 10% fetal bovine serum (FBS), 10 mM L-glutamine, 5 mM sodium pyruvate, antibiotic-antimycotic solution, and 5x nonessential amino acids). Cells from the Cantell strain of Sendai virus (SenV) were maintained according to accepted protocols. For assays, the cells were mock treated, transfected with either PAMP or XRNA formulated in liposomes, transfected with constitutively active RIG-I (N-RIG) or infected with SenV at 100 HAU/ml for the indicated times before analyzing for cell death by incucyte analyses, caspase 3/7 activity, or harvesting cell lysates for immunoblot and qRT-PCR analyses (as described in Kentsis, A. & Borden, K.L. Construction of macromolecular assemblages in eukaryotic processes and their role in human disease: linking RINGs together. Curr Protein Pept Sci 1, 49-73 (2000), Diaz-Griffero, F., et al., A B-box 2 surface patch important for TRIM5alpha self-association, capsid binding avidity, and retrovirus restriction. J Virol 83, 10737-10751 (2009), each of which are incorporated herein by reference in their entireties). Inhibitors were used at the following concentrations: zVAD (SM-Biochemicals) used at 1:1000, TNFa (Peprotech) [0.5µL of TNF/well with 400µL of total media/well], and Vaccinia Virus B18R Carrier-Free Recombinant Protein (eBioscience) [1 µg/ml]. Inhibitors were added three hours prior to PAMP or X RNA transfection.

### Immunoblot analysis

Protein extracts for immunoblot analyses were prepared by lysing cells on ice in MCLB lysis buffer (50 mM Tris HCl [pH 7.5], 150 mM NaCl, 0.5% NP-40) supplemented with 1 µM okadaic acid, 1 µM phosphatase inhibitor cocktail II (Calbiochem), and 10 µM protease inhibitor (Sigma), followed by 4°C centrifugation at 16,000 x g for 10 min to clarify the lysate. Equivalent protein amounts were separated using SDS-polyacrylamide gel electrophoresis followed by immunoblotting. The following primary antibodies were used for immunoblot analyses: PARP (Cell Signaling Technologies), RIG-I Alme-1 (Adipogen), ISG56 (rb-a-IFIT1 #971 made at UT Southwestern Antibody Core facility), IFITM1 (Protein Tech), ISG15 (Cell Signaling Technologies), Actin (Millipore), pSTAT1 Y701 (Cell Signaling Technologies), FLAG (Sigma), V5 and (Life Technologies). Alexa Fluor 680/790 donkey anti-rabbit and donkey anti-mouse (Jackson Immunoresearch) were used as secondary antibodies.

### Immunoprecipitation

Following exogenous expression of FLAG-RIG-I and TRIM16-V5 full length or mutant constructs in PH5CH8 or 293 cells, cell extracts were immunoprecipitated using α-FLAG agarose beads (Sigma) and α-V5 conjugated agarose beads (Sigma) overnight at 4°C. After immunoprecipitation, samples were washed three times using MCLB lysis buffer before elution. The eluate was boiled for 5 minutes and separated using SDS gel electrophoresis and analyzed by immunoblotting. α-V5 (Life Technologies) and α-FLAG (Sigma) were used as primary antibodies and were incubated overnight. Alexa Fluor 680/790 donkey anti-rabbit and donkey anti-mouse (Jackson Immunoresearch) were used as secondary antibodies.

### Cell death analysis

Cells were seeded on 24-well dishes at either 100 k cells per well. Cells were treated with 100 nM Sytox (Sytox) (#S7020, Thermo Fischer) to assay cell death and/or permeabilization or in separate wells with Syto-Green (S7559, Thermo Fischer) to assay total cell number. Cells were imaged with the IncuCyte imaging platform (Essen Bioscience). At each time point, nine images were taken per well. Each treatment was run in either duplicate or triplicate. Percent cell death was calculated by averaging Syto-Green counts over two hours near the beginning of the run. These were averaged with replicates and used to normalize Sytox counts to calculate percent cell death.

### Caspase 3/7 assay

Caspase 3/7 activity was measured using the Caspase 3/7 Green Apoptosis Assay Reagent (Essen) on the IncuCyte imaging platform (described above) and the Caspase-Glo^{®} 3/7 Assay System (Promega). Use and analysis were conducted according to manufacturer's instructions.

### Constructs and Cell transfection

RIG-I truncation mutant constructs were previously produced and described (PMID: 16585524, PMID: 17190814). TRIM16 truncation mutants were produced using a PCR-based cloning strategy. Oligonucleotide sequences used for TRIM16 cDNA production are available upon request. Cells were transfected with 2 µg/mL PAMP-RNA ¹⁰ as described (PMID: 18548002), control XRNA (5'ppp-containing RNA motif derived from the hepatitis C virus genome adjacent to and equivalent in length to PAMP-RNA but does not bind to RIG-I nor induce RIG-I signaling ¹⁰, cDNA constructs encoding TRIM16 (full length or truncated mutants), or RIG-I (full length or truncated mutant) using the *Trans*IT^{®}-mRNA Transfection kit (Mirus Bio LLC).

### RT-qPCR

Cells were harvested in RLT buffer (Qiagen) and total cellular RNA was purified using the RNeasy kit (Qiagen). cDNA was synthesized from the purified RNA by both random and oligo(dT) priming using the iScript cDNA synthesis kit (Bio-rad). RNA levels were measured by the SYBR Green relative quantitation method using either a 7300 or Viia 7 RT-PCR machine (Applied Biosystems). Samples were normalized by subtracting the respective CT values of housekeeping genes RPLPO or POLRA, and fold induction of specific genes calculated relative to untreated control. Primer sequences are listed in Table 1.

### Generation of Knockdown cell lines

Huh7 cells expressing CRISPR and non-targeting guide RNA (control) or guide RNA for knockout of RIG-I or the IFN-receptor chain (IFNAR) were described previously (PMID: 27841874). Huh7 cells expressing CRISPR and guide RNA specific to TRIM16 or TRIM25 were selected using the following set of guide RNAs to produce each specific knockdown knockout cell population:
TRIM16 guide RNAs 5' to 3'; each targets exon 6
GTCGGTGTCAGAGGTCAAAG (SEQ ID NO:)
GGTGTCAGAGGTCAAAGCGG (SEQ ID NO:)
GTCGGTGTCAGAGGTCAAAG (SEQ ID NO:)
TRIM25 guide RNAs 5' to 3'; each targets exon 3
GATGACTGCAAACAGAAAGG (SEQ ID NO:)
TCAGATGACTGCAAACAGAA (SEQ ID NO:)
GCAGCTACCAACAAGAATACA (SEQ ID NO:)

Typically, 3 independent gRNAs were selected per gene. gRNA sequences were subcloned into the pRRL-empty-gRNA-Cas9-T2A-Puro vector³² via InFusion cloning (TaKaRa). Constructs were transduced into Huh7 cells by spin-fection and polyclonal populations were isolated by Puromycin selection. Puromycin-resistant cells were then screened by RNA and immunoblot analyses to verify that the gene of interest was sufficiently knocked out.

### Proteomics analysis

PH5CH8 cells were transfected with full length FLAG-RIG-I as described above and either mock infected or infected with Sendai virus to activate RIG-I (100 HAU/mL) for 16 hours. Cells were then harvested to produce protein extracts. 200 ug of each protein extract were mixed with anti-FLAG-affinity beads (Sigma) and incubated at 4°C for 2 hrs. Beads were then washed 3-times in Tris buffered saline containing 150 mM NaCl followed by three additional washes in Tris buffered saline containing 250 mM NaCl. Bound proteins were then eluted through incubation of the beads with excess FLAG peptide. Eluate solution was then subjected to tandem liquid chromatography mass spectrometry (LC-MS/MS) analyses for identification of RIG-I and RIG-I-protein complexes. Five unique peptides for TRIM16 were identified.

### Statistical analysis

Statistical analyses were performed using GraphPad Prism 6 software (GraphPad, La Jolla, CA). Depending on the number of variables and time points in each experiment, statistical analysis of mean differences between groups was performed by either a Student's t-test or a multiway analysis of variance (ANOVA) followed by a Bonferroni *post hoc* analysis. Kaplan-Meier survival analyses were analyzed by the log-rank test. A comparison of mean survival and clinical symptoms were analyzed by the unpaired t-test. Specific statistical tests, *P* values and sample size are indicated in Figure Legends.

## Claims

1. A composition for use in the treatment of cancer in a subject, the composition comprising a pathogen-associated molecular pattern (PAMP)-containing nucleic acid molecule, wherein the PAMP-containing nucleic acid molecule comprises:
a 5'-arm region comprising a terminal triphosphate,
a poly-uracil core comprising at least 8 contiguous uracil residues, and
a 3'-arm region comprising at least 8 nucleic acid residues, wherein the 5'-most nucleic acid residue of the 3'-arm region is not a uracil and wherein the 3'-arm region is at least 30% uracil residues.

2. The composition of Claim 1, for use according to Claim 1, wherein:
(a) the poly-uracil core consists of between 8 and 30 uracil residues,
(b) the 5'-most nucleic acid residue of the 3'-arm region is a cytosine residue or a guanine residue,
(c) the 3'-arm region is at least 40%, 50%, 60%, 70%, 80%, or 90% uracil residues,
(d) the 3'-arm region comprises at least 7 contiguous uracil residues, and/or
(e) the 5'-arm region further comprises one or more nucleic acid residues disposed between the terminal triphosphate and the poly-uracil core, or the 5'-arm region consists of the terminal triphosphate and wherein the terminal triphosphate is linked directly to the 5'-end of the poly-uracil core,
in any combination.

3. The composition of Claim 1, for use according to Claim 1, wherein the PAMP-containing nucleic acid molecule induces retinoic acid-inducible gene I (RIG-I) like receptor (RLR) interaction with TRIM16 in a cancer cell in the subject, optionally wherein the RLR interaction with TRIM16 induces cell death signaling in the cancer cell, optionally wherein the RLR is RIG-I.

4. The composition of Claim 1, for use according to Claim 1, further comprising:
(a) interferon (IFN), and/or.
(b) a nucleic acid comprising a sequence encoding TRIM16 operatively linked to a promoter sequence, wherein the promoter sequence is capable of inducing expression of the encoded TRIM16 in a cancer cell within the subject, optionally wherein the encoded TRIM16 has an amino acid sequence of at least 90% identity to the amino acid sequence set forth in SEQ ID NO: 1.

5. The composition of Claim 1, for use according to Claim 1, wherein the cancer is a solid tumor cancer selected from pancreatic cancer, bladder cancer, colorectal cancer, breast cancer, prostate cancer, renal cancer, hepatocellular cancer, lung cancer, ovarian cancer, cervical cancer, gastric cancer, esophageal cancer, head and neck cancer, melanoma, neuroendocrine cancers, CNS cancers, brain tumors, bone cancer, and soft tissue sarcoma, or is a hematological cancer.

6. The composition of Claim 1, for use according to Claim 1, formulated to be administered in connection with an immunotherapy for the cancer, optionally wherein the immunotherapy comprises cancer-specific antibodies or functional fragments thereof, immune checkpoint inhibitors, and adoptive cell therapies, including CAR T-cell therapy.

7. The composition of Claim 1, for use according to Claim 1, wherein the composition is for use in the treatment of cancer in a human.

8. The composition of Claim 1, for use according to Claim 1, wherein the cancer is a hematological cancer.

9. The composition of Claim 1 for use according to Claim 1, comprising an immunotherapy for the cancer.

10. The composition of Claim 1, for use according to Claim 1, wherein the PAMP-containing nucleic acid molecule comprises a sequence of SEQ ID NO: 2.

11. The composition of Claim 1, for use according to Claim 1, wherein the PAMP-containing nucleic acid molecule consists of a sequence of SEQ ID NO: 2.

## Patentansprüche

1. Zusammensetzung zur Verwendung in der Behandlung von Krebs bei einer Person, wobei die Zusammensetzung ein Nukleinsäuremolekül umfasst, das ein pathogenassoziiertes molekulares Muster (PAMP) enthält, wobei das PAMP-haltige Nukleinsäuremolekül umfasst:
eine 5'-Armregion, die ein terminales Triphosphat umfasst,
einen Polyuracil-Kern, der mindestens 8 zusammenhängende Uracilreste umfasst, und
eine 3'-Armregion, die mindestens 8 Nukleinsäurereste umfasst, wobei der 5' am weitesten entfernte Nukleinsäurerest der 3'-Armregion kein Uracil ist und wobei die 3'-Armregion zu mindestens 30 % Uracilresten entspricht.

2. Zusammensetzung nach Anspruch 1 zur Verwendung nach Anspruch 1, wobei:
a) der Polyuracil-Kern aus zwischen 8 und 30 Uracilresten besteht,
b) der 5' am weitesten entfernte Nukleinsäurerest der 3'-Armregion ein Cytosinrest oder ein Guaninrest ist,
c) die 3'-Armregion zu mindestens 40 %, 50 %, 60 %, 70 %, 80 %, oder 90 % Uracilresten entspricht,
d) die 3'-Armregion mindestens 7 zusammenhängende Uracilreste umfasst, und/oder
e) die 5'-Armregion weiter einen oder mehrere Nukleinsäurereste umfasst, die zwischen dem terminalen Triphosphat und dem Polyuracil-Kern angeordnet sind, oder die 5'-Armregion aus dem terminalen Triphosphat besteht, und wobei das terminale Triphosphat direkt mit dem 5'-Ende des Polyuracil-Kerns verknüpft ist,
in beliebiger Kombination.

3. Zusammensetzung nach Anspruch 1 zur Verwendung nach Anspruch 1, wobei das PAMP-haltige Nukleinsäuremolekül Interaktion des Retinsäure-induzierbaren Gen I- (RIG-I-) ähnlichen Rezeptors (RLR) mitTRIM16 in einer Krebszelle in der Person induziert, wobei die RLR-Interaktion mit TRIM16 optional Zelltodsignalisierung in der Krebszelle induziert, wobei der RLR optional RIG-I ist.

4. Zusammensetzung nach Anspruch 1 zur Verwendung nach Anspruch 1, weiter umfassend:
a) Interferon (IFN), und/oder
b) eine Nukleinsäure, die eine TRIM16 codierende Sequenz umfasst, die operativ mit einer Promotorsequenz verknüpft ist, wobei die Promotorsequenz in der Lage ist, Expression des codierten TRIM16 in einer Krebszelle innerhalb der Person zu induzieren, wobei das codierte TRIM16 optional eine Aminosäuresequenz mit mindestens 90 % Identität mit der in SEQ. ID-Nr. 1 dargelegten Aminosäuresequenz aufweist.

5. Zusammensetzung nach Anspruch 1 zur Verwendung nach Anspruch 1, wobei der Krebs ein solider Tumor ist, ausgewählt aus Bauchspeicheldrüsenkrebs, Blasenkrebs, Kolorektalkrebs, Brustkrebs, Prostatakrebs, Nierenkrebs, Leberzellkrebs, Lungenkrebs, Eierstockkrebs, Gebärmutterhalskrebs, Magenkrebs, Speiseröhrenkrebs, Kopf- und Halskrebs, Melanom, neuroendokrinen Krebsen, ZNS-Krebsen, Gehirntumoren, Knochenkrebs und Weichteilsarkom, oder ein Blutkrebs ist.

6. Zusammensetzung nach Anspruch 1 zur Verwendung nach Anspruch 1, die formuliert ist, um in Verbindung mit einer Immuntherapie gegen den Krebs verabreicht zu werden, wobei die Immuntherapie optional krebsspezifische Antikörper oder funktionelle Fragmente davon, Immun-Checkpoint-Inhibitoren und adoptive Zelltherapien, die CAR-T-Zelltherapie beinhalten, umfasst.

7. Zusammensetzung nach Anspruch 1 zur Verwendung nach Anspruch 1, wobei die Zusammensetzung zur Verwendung bei der Behandlung von Krebs bei einem Menschen bestimmt ist.

8. Zusammensetzung nach Anspruch 1 zur Verwendung nach Anspruch 1, wobei der Krebs ein Blutkrebs ist.

9. Zusammensetzung nach Anspruch 1 zur Verwendung nach Anspruch 1, die eine Immuntherapie gegen den Krebs umfasst.

10. Zusammensetzung nach Anspruch 1 zur Verwendung nach Anspruch 1, wobei das PAMP-haltige Nukleinsäuremolekül eine Sequenz von SEQ. ID-Nr. 2 umfasst.

11. Zusammensetzung nach Anspruch 1 zur Verwendung nach Anspruch 1, wobei das PAMP-haltige Nukleinsäuremolekül aus einer Sequenz von SEQ. ID-Nr. 2 besteht.

## Revendications

1. Composition destinée à être utilisée dans le traitement du cancer chez un sujet, la composition comprenant une molécule d'acide nucléique contenant un motif moléculaire associé à un pathogène (PAMP), dans laquelle la molécule d'acide nucléique contenant un PAMP comprend :
une région du bras 5' comprenant un triphosphate terminal,
un noyau poly-uracile comprenant au moins 8 résidus uracile contigus, et
une région du bras 3' comprenant au moins 8 résidus acide nucléique, dans laquelle le résidu d'acide nucléique de dernière base en 5' de la région du bras 3' n'est pas un uracile et dans laquelle la région du bras 3' est constituée d'au moins 30 % de résidus uracile.

2. Composition selon la revendication 1, destinée à être utilisée selon la revendication 1, dans laquelle :
a) le noyau poly-uracile consiste en 8 à 30 résidus uracile,
b) le résidu d'acide nucléique de dernière base en 5' de la région du bras 3' est un résidu cytosine ou un résidu guanine,
c) la région du bras 3' est au moins 40 %, 50 %, 60 %, 70 %, 80 % ou 90 % de résidus uracile,
d) la région du bras 3' comprend au moins 7 résidus uracile contigus, et/ou
e) la région du bras 5' comprend en outre un ou plusieurs résidus acide nucléique disposés entre le triphosphate terminal et le noyau poly-uracile, ou la région du bras 5' consiste en le triphosphate terminal et dans laquelle le triphosphate terminal est lié directement à l'extrémité 5' du noyau poly-uracile, dans n'importe quelle combinaison.

3. Composition selon la revendication 1, destinée à être utilisée selon la revendication 1, dans laquelle la molécule d'acide nucléique contenant un PAMP induit une interaction du récepteur (RLR) de type gène I inductible par l'acide rétinoïque (RIG-I) avec TRIM16 dans une cellule cancéreuse chez le sujet, facultativement, dans laquelle l'interaction de RLR avec TRIM16 induit une signalisation de mort cellulaire dans la cellule cancéreuse, facultativement dans laquelle le RLR est RIG-I.

4. Composition selon la revendication 1, destinée à être utilisée selon la revendication 1, comprenant en outre :
a) un interféron (IFN), et/ou
b) un acide nucléique comprenant une séquence codant pour TRIM16 liée de manière fonctionnelle à une séquence promotrice, dans laquelle la séquence promotrice permet d'induire l'expression du TRIM16 codé dans une cellule cancéreuse chez le sujet, facultativement dans laquelle le TRIM16 codé présente une séquence d'acides aminés d'au moins 90 % d'identité avec la séquence d'acides aminés présentée dans SEQ ID NO : 1.

5. Composition selon la revendication 1, destinée à être utilisée selon la revendication 1, dans laquelle le cancer est un cancer à tumeur solide choisi parmi le cancer du pancréas, le cancer de la vessie, le cancer colorectal, le cancer du sein, le cancer de la prostate, le cancer du rein, le cancer hépatocellulaire, le cancer du poumon, le cancer des ovaires, le cancer du col de l'utérus, le cancer gastrique, le cancer de l'oesophage, le cancer de la tête et du cou, un mélanome, des cancers neuroendocriniens, des cancers du SNC, des tumeurs cérébrales, le cancer des os et un sarcome des tissus mous ou est un cancer hématologique.

6. Composition selon la revendication 1, destinée à être utilisée selon la revendication 1, formulée pour être administrée en relation avec une immunothérapie pour le cancer, facultativement dans laquelle l'immunothérapie comprend des anticorps spécifiques du cancer ou des fragments fonctionnels de ceux-ci, des inhibiteurs de point de contrôle immunitaire et des thérapies cellulaires adoptives, incluant une thérapie CAR-T.

7. Composition selon la revendication 1, destinée à être utilisée selon la revendication 1, dans laquelle la composition est destinée à être utilisée dans le traitement du cancer chez un être humain.

8. Composition selon la revendication 1, destinée à être utilisée selon la revendication 1, dans laquelle le cancer est un cancer hématologique.

9. Composition selon la revendication 1, destinée à être utilisée selon la revendication 1, comprenant une immunothérapie pour le cancer.

10. Composition selon la revendication 1, destinée à être utilisée selon la revendication 1, dans laquelle la molécule d'acide nucléique contenant un PAMP comprend une séquence de SEQ ID NO : 2.

11. Composition selon la revendication 1, destinée à être utilisée selon la revendication 1, dans laquelle la molécule d'acide nucléique contenant un PAMP consiste en une séquence de SEQ ID NO : 2.
